(19) Europäisches Patentamt
European Patent Office
Office européen des brevets

(11) **EP 4 306 136 A1**

(12) **EUROPEAN PATENT APPLICATION**
published in accordance with Art. 153(4) EPC

(43) Date of publication:
**17.01.2024 Bulletin 2024/03**

(21) Application number: **22766639.3**

(22) Date of filing: **24.01.2022**

(51) International Patent Classification (IPC):
**A61L 2/10** *(2006.01)* **A61L 9/20** *(2006.01)*

(52) Cooperative Patent Classification (CPC):
**A61L 2/10; A61L 9/20**

(86) International application number:
**PCT/JP2022/002380**

(87) International publication number:
**WO 2022/190672 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.03.2021 JP 2021037424**
**26.10.2021 JP 2021174849**

(71) Applicant: **Ushio Denki Kabushiki Kaisha**
**Tokyo 100-8150 (JP)**

(72) Inventors:
• **NAITO, Keisuke**
**Tokyo 100-8150 (JP)**
• **IGARASHI, Tatsushi**
**Tokyo 100-8150 (JP)**
• **FUJINA, Kyosuke**
**Tokyo 100-8150 (JP)**

(74) Representative: **Maiwald GmbH**
**Engineering**
**Elisenhof**
**Elisenstrasse 3**
**80335 München (DE)**

(54) **APPARATUS FOR INACTIVATING BACTERIA OR VIRUSES, AND SYSTEM FOR INACTIVATING BACTERIA OR VIRUSES**

(57) An apparatus for inactivating bacteria or viruses in a space, the apparatus includes a light source unit that emits ultraviolet light having a peak wavelength within a wavelength range of 190 nm or more and less than 240 nm, an irradiation region changing mechanism that changes an irradiation direction of the ultraviolet light of the light source unit, and a driving unit that drives the irradiation region changing mechanism to change the irradiation direction of the ultraviolet light of the light source unit.

**Fig.1**

## Description

TECHNICAL FIELD

The present invention relates to an apparatus for inactivating bacteria or viruses, and in particular, an apparatus for inactivating bacteria or viruses by using ultraviolet light. The present invention also relates to a system for inactivating bacteria or viruses.

BACKGROUND ART

Conventionally, a technology for inactivating bacteria or viruses by irradiating bacteria or viruses with ultraviolet light has been known. DNA exhibits the highest absorption characteristics around a wavelength of 260 nm, and therefore in many cases, ultraviolet light that is emitted from a light source such as a low-pressure mercury lamp and has a wavelength of around 254 nm is used. A method for inactivating bacteria or viruses by using ultraviolet light is characterized by being able to perform sterilization treatment by only irradiating a space to be treated or an object to be treated with ultraviolet light without spraying chemicals or the like.

However, it is known that irradiation of a human body with ultraviolet light in a specified wavelength band has a risk of affecting the human body. Therefore, a method or an inactivation apparatus for inactivating bacteria or viruses in a space without irradiating humans with ultraviolet light has been examined.

For example, Patent Document 1 listed below describes an inactivation apparatus that normally emits ultraviolet light near a ceiling in a space, and also emits ultraviolet light for inactivation treatment to a lower region in the space where humans are expected to come and go only in a case where no humans are present in the space. Furthermore, Patent Document 2 listed below describes an inactivation apparatus that changes a wavelength of emitted light depending on the presence or absence of a human in a space.

PRIOR ART DOCUMENT

PATENT DOCUMENTS

Patent Document 1: JP-A-2018-130535
Patent Document 2: US 10,588,993

SUMMARY OF THE INVENTION

PROBLEMS TO BE SOLVED BY THE INVENTION

For example, in general living spaces, working spaces, or the like, bacteria or viruses adhere to aerosol, dust, or the like, and float. Not a few bacteria or viruses adhere to everything including the ceiling, walls, the floor, a desk or a chair disposed in the space, and the like.

However, the inactivation apparatus described in Patent Document 1 listed above performs control to turn on or off an ultraviolet light source for inactivation treatment depending on the presence of a human, but does not perform control to change a direction of irradiation with ultraviolet light. Therefore, in a space where inactivation treatment is performed, a region that is irradiated with ultraviolet light emitted from the inactivation apparatus is limited to a partial region of the space. Accordingly, the inactivation apparatus described in Patent Document 1 listed above will never perform inactivation treatment on bacteria or viruses that adhere to a region that is not irradiated with ultraviolet light in the space.

Patent Document 2 listed above discloses a configuration in which a direction of irradiation with ultraviolet light can be adjusted after installation. However, in the configuration described in Patent Document 2 listed above, if an attempt is made to irradiate a desired region with ultraviolet light, an operator needs to perform a task of appropriately adjusting a direction of irradiation with ultraviolet light in consideration of safety.

Furthermore, in the case of a conventionally used inactivation apparatus that uses ultraviolet light of around 254 nm, if an operator approaches the inactivation apparatus, and makes an attempt to adjust a direction of irradiation with ultraviolet light, there is a possibility that the operator will be irradiated with ultraviolet light that affects the human body.

In view of the problems described above, an object of the present invention is to provide an apparatus for inactivating bacteria or viruses and a system for inactivating bacteria or viruses that are capable of irradiating a wide range in a space with ultraviolet light that can inactivate bacteria or viruses, and have improved safety.

MEANS FOR SOLVING THE PROBLEMS

An apparatus for inactivating bacteria or viruses according to the present invention is
an apparatus for inactivating bacteria or viruses in a space, the apparatus including:

a light source unit that emits ultraviolet light having a peak wavelength within a wavelength range of 190 nm or more and less than 240 nm;
an irradiation region changing mechanism that changes an irradiation direction of the ultraviolet light of the light source unit; and
a driving unit that drives the irradiation region changing mechanism to change the irradiation direction of the ultraviolet light of the light source unit.

Herein, "inactivation" refers to a concept that includes killing bacteria or viruses or making infectivity or toxicity lost, and "bacteria" refer to microorganisms such as germs or fungi (mold). Hereinafter, in some cases, the "bacteria or viruses" are collectively referred to as "pathogens".

The present inventors have made intensive studies to discover that light within a wavelength range of 190 or more and less than 240 nm has high safety to humans or animals, and can kill microorganisms or can inactivate viruses.

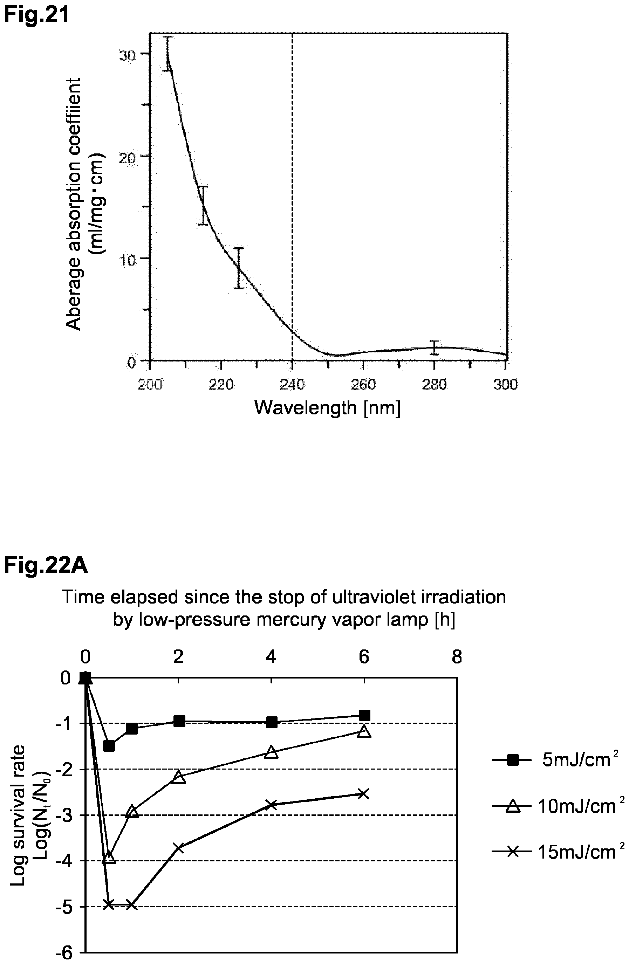

Fig. 21 is a graph indicating a characteristic of an average absorption coefficient in an ultraviolet light region of protein. As illustrated in Fig. 21, it is apparent that protein is difficult to absorb ultraviolet light having a wavelength of 250 nm or more, but protein sharply becomes easy to absorb ultraviolet light in a wavelength band that is shorter than a wavelength of 240 nm. Stated another way, ultraviolet light having a wavelength of 240 nm or more is easily transmitted through human skin, and permeates through an inside of skin. Therefore, cells inside human skin are easily damaged. In contrast, ultraviolet light having a wavelength of less than 240 nm is easy to be absorbed on a surface (for example, stratum corneum) of human skin, and is difficult to permeate through an inside of skin. Therefore, ultraviolet light having a wavelength of less than 240 nm is safe to skin.

On the other hand, if ultraviolet light having a wavelength of less than 190 nm is present, oxygen molecules present in the atmosphere are photolyzed to generate many oxygen atoms, and the oxygen molecule and the oxygen atom react together by bonding to generate much ozone. Therefore, it is undesirable to apply ultraviolet light having a wavelength of less than 190 nm in the atmosphere.

Accordingly, it can be said that ultraviolet light within a wavelength range of 190 nm or more and less than 240 nm is ultraviolet light having high safety to humans or animals. From a viewpoint of improvements in safety to humans or animals, it is preferable that ultraviolet light emitted from a light source unit be within a wavelength range of 190 nm or more and less than 237 nm, it is more preferable that the ultraviolet light be within a wavelength range of 190 nm or more and less than 235 nm, and it is particularly preferable that the ultraviolet light be within a wavelength range of 190 nm or more and less than 230 nm.

Furthermore, ultraviolet light having a wavelength of 240 nm or more and less than 280 nm is harmful to humans or animals, and therefore in order to improve safety, it is desirable that the light intensity of light emitted from the light source unit be reduced in a wavelength range of 240 nm or more and less than 280 nm. Specifically, it is preferable that a light intensity in the wavelength range be reduced to less than 5% relative to a light intensity at a peak wavelength, it is more preferable that the light intensity be reduced to less than 3%, and it is particularly preferable that the light intensity be reduced to less than 1%. As the light source unit, a light source having a light emission spectrum that satisfies the wavelength range described above can be selected, or a light source in which a wavelength range of ultraviolet light to be emitted has been adjusted by separately using an optical filter or the like can be used.

A product covered by the present invention can provide sterilization and virus inactivation performance intrinsic to ultraviolet light without causing erythema or keratitis on the skin or eyes of a human or an animal. In particular, taking advantage of a characteristic of being able to be used in an environment where a human is present in contrast to conventional ultraviolet light sources, the product can be installed in such an environment indoors or outdoors to irradiate the entire environment and provide virus inhibition and bacteria elimination in the air and on a surface of members installed in the environment.

This accords with Goal 3 "Ensure healthy lives and promote well-being for all at all ages" included in the Sustainable Development Goals (SDGs) led by the United Nations, and will greatly contribute to the goal target 3.3 "By 2030, end the epidemics of AIDS, tuberculosis, malaria and neglected tropical diseases and combat hepatitis, water-borne diseases and other communicable diseases".

Furthermore, as described above, ultraviolet light having a wavelength of less than 190 nm is absorbed into oxygen in the air, and this results in generation of ozone. In order to more effectively inhibit ozone from being generated, as described above, it is desirable that ultraviolet light having a peak wavelength of 200 nm or more be used. Therefore, it is preferable that a peak wavelength of ultraviolet light emitted from the light source unit be within a wavelength range of 200 nm or more and less than 237 nm, it is more preferable that the peak wavelength be within a wavelength range of 200 nm or more and less than 235 nm, and it is further preferable that the peak wavelength be within a wavelength range of 200 nm or more and less than 230 nm.

Examples of a light source that emits such light include an excimer lamp in which KrCl has been sealed as light-emitting gas into a tube body, and that emits ultraviolet light having a peak wavelength of 222 nm, and an excimer lamp in which KrCl has been sealed as light-emitting gas into a tube body, and that emits ultraviolet light having a peak wavelength of 207 nm. Stated another way, both ultraviolet light that is emitted from a KrCl excimer lamp and has a peak wavelength of 222 nm or ultraviolet light that is emitted from a KrBr excimer lamp and has a peak wavelength of 207 nm are light that is safe to humans or animals, and can kill microorganisms or can inactivate viruses. Therefore, even if a human or an animal is present in a sterilized or inactivated region in a space, a sterilization or inactivation task can be performed by performing irradiation with ultraviolet light.

Furthermore, a solid-state light source such as an LED or an LD may be used. For example, as an LED having a light emission region at a wavelength of 190 nm or more and less than 240 nm, an aluminum gallium nitride (AlGaN) based light-emitting element, an aluminum nitride (AlN) based light-emitting element, or a zinc magnesium oxide (MgZnO) based light-emitting element can be employed. Moreover, a combination with a wavelength conversion element may be employed. For example, ultraviolet light having a wavelength of 190 nm or more and less than 240 nm may be generated by using, as a wavelength conversion element, non-linear optical crystal that multiplies a frequency of light emitted from a gas laser or a solid-state laser element to cause high harmonic generation such as second harmonic generation (SHG) or third harmonic generation (THG).

Moreover, the inactivation apparatus according to the present invention includes the irradiation region changing mechanism that changes an irradiation direction of ultraviolet light to be emitted from the light source unit, and the driving unit that drives the irradiation region changing mechanism. This enables inactivation treatment to be performed on a surface of a target object or a target space while varying the irradiation position of ultraviolet light. For example, the irradiation position is varied with the lapse of time, and this enables inactivation treatment to be sequentially performed on a wide space or a plurality of positions. As an operation performed by the driving unit to drive the irradiation region changing mechanism, the irradiation region changing mechanism may be driven in such a way that the irradiation position of ultraviolet light is always varied, or may be driven in such a way that a variation and a stop are repeated.

Moreover, irradiation with ultraviolet light within a wavelength range of 190 nm and more and less than 240 nm enables inactivation treatment to be effectively performed, even if the irradiation position of ultraviolet light varies. Specifically, some bacteria contain an enzyme that repairs damage to DNA caused by ultraviolet light. An example of the enzyme that repairs damage to DNA in bacteria is flavin adenine dinucleotide (FAD), and irradiation with light having a wavelength of 300 nm to 500 nm causes FAD to exhibit an effect of repairing damage to DNA in bacteria. A phenomenon in which damage to DNA in bacteria is recovered from as a result of irradiation of a specified enzyme with light within a predetermined wavelength range, as described above, is referred to as "photoreactivation of bacteria" in some cases.

In a case where inactivation is performed by using conventional ultraviolet light of 254 nm, which is known as bactericidal rays, if there is time when irradiation with ultraviolet light is not performed, bacteria easily proliferate due to photoreactivation, and it is difficult to effectively achieve inactivation. Stated another way, if an attempt to be made to perform inactivation treatment on a surface of an irradiation target object in such a way that photoreactivation is not caused, it is requested that irradiation with ultraviolet light be continued as much as possible, and it is difficult to advance inactivation treatment while varying the irradiation position of ultraviolet light.

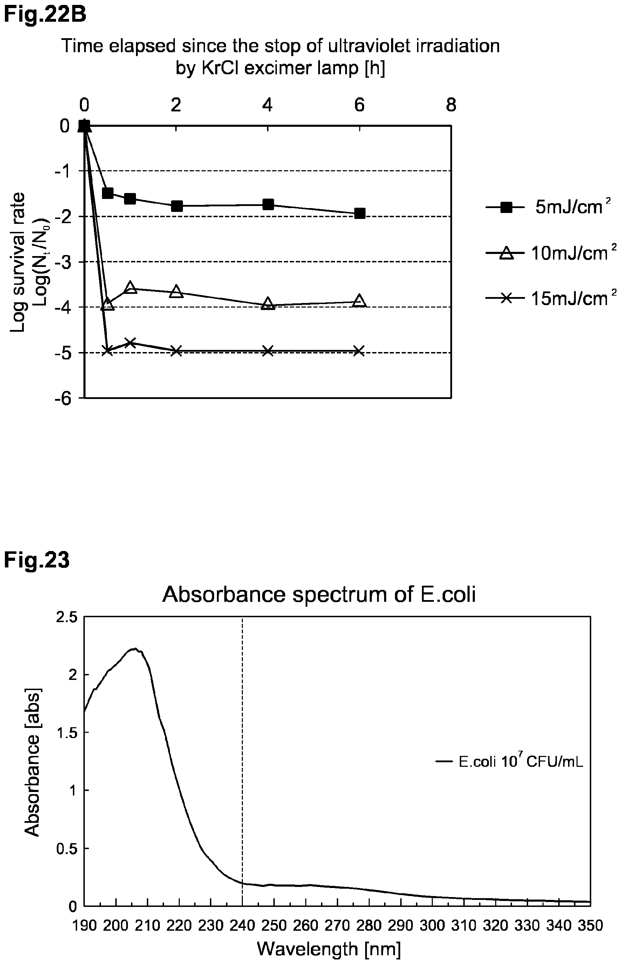

In contrast, it has been discovered that an effect of inhibiting "photoreactivation of bacteria" is exhibited in a wavelength range of less than 240 nm. Fig. 22A is a graph indicating a time change in a survival rate after irradiation of Staphylococcus aureus with ultraviolet light containing photolyase, by using a low-pressure mercury lamp that emits ultraviolet light having a peak wavelength of 254 nm, and Fig. 22B is a graph indicating a time change in a survival rate after irradiation of Staphylococcus aureus containing photolyase with ultraviolet light, by using an excimer lamp that emits ultraviolet light having a peak wavelength of 222 nm and in which KrCl has been sealed as light-emitting gas into a tube body. Both graphs indicate a result of performing irradiation with ultraviolet light for 30 minutes in an environment of irradiation with visible light, stopping irradiation with ultraviolet light, and checking a change in the number of existence of bacteria. Furthermore, the cases of different irradiance of ultraviolet light were verified in order to make a comparison among three types of amounts of irradiation with ultraviolet light, 5 mJ/cm$^2$, 10 mJ/cm$^2$, and 15 mJ/cm$^2$. Note that a vertical axis corresponds to a log value of a ratio of the number of colonies ($N_t$) of Staphylococcus aureus after irradiation with ultraviolet light L1 by using, as a reference, the number of colonies ($N_0$) of Staphylococcus aureus before irradiation.

As is apparent from the graphs illustrated in Figs. 22A and 22B, photoreactivation of bacteria itself is inhibited in a case where ultraviolet light having a wavelength of 222 nm is used. It can be considered that a wavelength range of less than 240 nm effectively acts on cellular tissue that forms bacteria or viruses, and this inhibited a function of photoreactivation.

As illustrated in Fig. 21, it is apparent that an average absorption coefficient for protein increases in a wavelength band that is shorter than 240 nm. Therefore, ultraviolet light is effectively absorbed into protein, which is a component of cell membrane or an enzyme contained in bacteria or viruses. In particular, ultraviolet light in a wavelength band that is shorter than 240 nm is absorbed on a surface (for example, stratum corneum) of human skin, is difficult to permeate through an inside of skin, and is highly safe to skin. However, bacteria or viruses are much smaller physically than human cells, and even ultraviolet light in a wavelength band that is shorter than 240 nm easily reaches inside. Therefore, it can be considered that ultraviolet light in a wavelength band that is shorter than 240 nm effectively acts on cells that form bacteria or viruses and in particular, cell membrane, an enzyme, or the like containing a protein component, and an effect of inhibiting a function of photoreactivation or the like of bacteria is enhanced.

Here, in order to check absorption characteristics for protein, the absorbance of adjusted Escherichia coli undiluted solution was measured. A method for measuring absorbance and a method for adjusting Escherichia coli undiluted solution are described below.

Escherichia coli (NBRC.106373 freeze-dried product) was suspended in an LB medium, and shaking culture was performed at 37°C for 24 hours. Moreover, the suspension described above was diluted with the LB medium to 1/10$^5$ to 1/10$^7$, was smeared by 0.1 mL on a standard agar medium, and was cultivated at 37°C for 24 hours. Moreover, one colony was bacteria-collected from the standard agar medium of 30 to 300 CFU/Plate, by using a platinum loop, was suspended in an LB medium of 5 mL, and shaking culture was performed at 37°C for 4 hours. Centrifugal washing was performed on the suspension described above by using physiological saline solution, and Escherichia coli undiluted solution was prepared. The concentration of the undiluted solution obtained as a result of the task described above is $10^9$ CFU/mL. In measuring absorbance, reagent solution prepared by diluting the undiluted solution to 1/100 to have a concentration of $10^7$ CFU/mL was used, and NanoDrop from Thermo Fisher Science was used.

Fig. 23 is a graph indicating an absorption spectrum of Escherichia coli (E. Coli). As illustrated in Fig. 23, it is apparent that the absorbance of Escherichia coli (E. Coli) increases for light in a wavelength band that is shorter than 240 nm, similarly to a tendency of the average absorption coefficient for protein. This indicates that light in a wavelength band that is shorter than 240 nm effectively acts on cellular tissue that forms bacteria or viruses.

Therefore, in comparison with an inactivation apparatus using conventional ultraviolet light, ultraviolet light that belongs to a wavelength band of less than 240 nm inhibits photoreactivation of bacteria, and even intermittent irradiation with ultraviolet light exhibits a satisfactory inactivating effect. Stated another way, even in a case where inactivation is performed while varying the irradiation position of ultraviolet light, inactivation treatment can be efficiently performed. From a viewpoint of effectively inhibiting photoreactivation of bacteria, it is preferable that a peak wavelength of ultraviolet light emitted from the light source unit be within a wavelength range of 190 nm or more and less than 235 nm, and it is more preferable that the peak wavelength be within a wavelength range of 190 nm or more and less than 230 nm.

In the inactivation apparatus, the light source unit may include an optical filter configured to reduce a light intensity at least in a wavelength range of 240 nm or more and less than 280 nm.

Herein, to "reduce the light intensity" means that a light intensity is less than 5% relative to a light intensity at a peak wavelength, it is more preferable that the light intensity be less than 3%, and it is further preferable that the light intensity be less than 1%.

Ultraviolet light having a wavelength of 240 nm or more and less than 280 nm is harmful to humans or animals. Therefore, in order to improve safety, it is desirable that the light intensity of light emitted from the light source unit be reduced in a wavelength range of 240 nm or more and less than 280 nm. In reducing light intensity, it is preferable that a light intensity in the wavelength range be reduced to less than 5% relative to a light intensity at a peak wavelength, it is more preferable that the light intensity be reduced to less than 3%, and it is particularly preferable that the light intensity be reduced to less than 1%.

Furthermore, the KrCl excimer lamp or the KrBr excimer lamp has a peak wavelength within a wavelength range of 200 to 230 nm, but slightly includes ultraviolet light within a wavelength range of 240 nm or more and less than 280 nm. In such a case, in order to prevent irradiation with ultraviolet light of a wavelength of 240 nm or more and less than 280 nm, it is desirable that an optical filter be provided in the light source unit, as described above.

As the optical filter, for example, a wavelength selection filter that transmits light within a wavelength range of 200 nm or more and less than 237 nm, and cuts off light having a wavelength of 240 or more and less than 280 nm can be used. Here, as the wavelength selection filter, for example, a dielectric multilayer film filter including a HfOz layer and a SiOz layer can be used. As described above, by proving the optical filter in a light emission window, even in a case where light harmful to humans is slightly emitted from an excimer lamp, the light can be securely prevented from leaking to an outside of a housing.

Furthermore, in the inactivation apparatus, the driving unit may drive the irradiation region changing mechanism to cause the irradiation position of the ultraviolet light emitted from the light source unit to reciprocally move in a predetermined direction or move around through a predetermined irradiation route.

By employing the configuration described above, irradiation with ultraviolet light in a predetermined irradiation position is intermittently continued, and this can advance inactivation treatment. In comparison with a conventional inactivation apparatus in which an irradiation region is not varied, inactivation treatment can be performed over a wide range in a space.

The inactivation apparatus may include

a pattern storage that stores data of an operation pattern of the irradiation region changing mechanism, and
the driving unit may drive the irradiation region changing mechanism on the basis of the data stored in the pattern storage.

By employing the configuration described above, a direction of irradiation with ultraviolet light of the light source unit automatically changes on the basis of the data of the operation pattern of the irradiation region changing mechanism that has been stored in advance in the pattern storage. Accordingly, the entirety in a space can be irradiated with ultraviolet light, or a region where inactivation treatment has not been sufficiently performed can be intensively irradiated with ultraviolet light.

Furthermore, from the pattern storage included in the inactivation apparatus according to the present invention, the operation pattern of the irradiation direction region mechanism that has been stored in advance may be only read, or the stored data may be rewritable in wired communication or wireless communication with an external device.

In the inactivation apparatus,
the driving unit may drive the irradiation region changing mechanism to switch a direction of irradiation with the ultraviolet light of the light source unit between a first space and a second space in the space, the first space is a space located at a height of less than 2 m from a floor, and the second space is a space located at a height of more than 2 m from the floor.

Herein, to "irradiate the first space with ultraviolet light" means that under the assumption that a principal ray indicating a highest intensity of ultraviolet light emitted from the light source unit advances straight in the space without disappearing, irradiation with the ultraviolet light is performed in such a way that the ultraviolet light reaches the floor or a wall in the first space. Furthermore, to "irradiate the second space with ultraviolet light" means that under the assumption that a principal ray of ultraviolet light emitted from the light source unit advances straight in the space without disappearing, irradiation with the ultraviolet light is performed in such a way that the ultraviolet light reaches the ceiling or a wall in the second space.

For example, in the case of irradiation with ultraviolet light toward the floor from the inactivation apparatus installed on the ceiling, a principal ray of the ultraviolet light always passes through the second space, and reaches the floor under the assumption that the principal ray advances straight in the space without disappearing. In such a case, the principal ray of the ultraviolet light passes through the second space, but the principal ray of the ultraviolet light that is assumed to advance straight in the space without disappearing reaches the floor. This means that "the first space is irradiated with ultraviolet light".

In the space, pathogens that adhere to disposed articles or the like, such as a desk or a chair, and pathogens that are floating in the space are present. The articles, such as a desk or a chair, that are normally used by a human are disposed in a region that is less than 2 m, which is roughly a region that is lower than the human's height, in the space. Stated another way, in a living space or the like, in a case where it is desired that inactivation treatment is performed on pathogens that adhere to the installed articles, it is requested that irradiation with ultraviolet light be performed toward the first space is a space located at a height of less than 2 m from the floor.

Pathogens that adhere to aerosol or the like and are floating in the space diffuse in the space in about several tens of seconds to several tens of minutes, and are convected between an upper region and a lower region of the space. Therefore, it can be considered effective to irradiate the first space and the second space with ultraviolet light.

In view of the above, in order to perform inactivation treatment in the space, it can be considered effective to perform irradiation with ultraviolet light while switching control to irradiate, with ultraviolet light, pathogens that adhere to articles or the like and control to irradiate, with ultraviolet light, pathogens that are floating in the space, without sequential irradiation with ultraviolet light from an end of the space. This effect will be confirmed referring to a calculation result in the item "MODE FOR CARRYING OUT THE INVENTION".

By employing the configuration described above, inactivation treatment can be more effectively on pathogens that are present in the space.

The inactivation apparatus may include

a first lighting controller that controls an emission intensity or a lighting time of the light source unit, and the first lighting controller may control the light source unit to cause an amount of irradiation per unit time of the first space with the ultraviolet light to be smaller than the amount of irradiation per unit time of the second space with the ultraviolet light.

Moreover, in the inactivation apparatus,
the first lighting controller may control the light source unit to cause the emission intensity of the light source unit at a time of irradiation of the first space with the ultraviolet light to be lower than the emission intensity of the light source unit at a time of irradiation of the second space with the ultraviolet light.

Furthermore, the inactivation apparatus may include

a timer that measures an irradiation time of the ultraviolet light, and
the driving unit may drive the irradiation region changing mechanism to switch the irradiation of the first space with the ultraviolet light and the irradiation of the second space with the ultraviolet light on the basis of a time measured by the timer.

Moreover, in the inactivation apparatus,
the driving unit may drive the irradiation region changing mechanism to cause a time during which the light source unit will irradiate the second space with the ultraviolet light to be longer than a time during which the light source unit has irradiated the first space with the ultraviolet light immediately before.

The inactivation apparatus may include

a human detecting sensor that senses whether a human is present in the space,
when the human detecting sensor has sensed presence of the human in the space, the driving unit may drive the irradiation region changing mechanism to cause the light source unit to irradiate the second space with the ultraviolet light, and
when the human detecting sensor has sensed absence of the human in the space, the driving unit may drive the irradiation region changing mechanism to cause the light source unit to irradiate the first space with the ultraviolet light.

Moreover, in the inactivation apparatus, the human detecting sensor may be provided to have a sensing region that is fixed.

At a point in time of the filing date of this application, a threshold limit value (TLV) for each wavelength of an amount of irradiation of a human body with ultraviolet light per day (8 hours) has been specified by American Conference of Governmental Industrial Hygienists (ACGIH), JIS Z 8812 (Measuring methods of eye-hazardous ultraviolet radiation), or the like. In other words, in a case where ultraviolet light is used in an environment where a human is present, it is recommended to determine an emission intensity or a lighting time of a light source unit in such a way that an integrated amount of irradiation of ultraviolet light applied during a predetermined time period is equal to or less than a reference value of TLV.

These specifications also specify a threshold limit value for ultraviolet light having a wavelength of 190 nm or more and less than 240 nm. Therefore, even in a case where inactivation treatment is performed by using ultraviolet light having a wavelength of 190 nm or more and less than 240 nm, which has an extremely small risk of affecting human bodies, it is preferable that humans be not irradiated with the ultraviolet light for a long time.

In view of this, the light source unit is controlled as described above, and this causes an integrated amount of irradiation of ultraviolet light applied to the first space where the presence of a human is expected to be lower than an integrated amount of irradiation of ultraviolet light applied to the second space. Accordingly, in a case where a human is present in the space, an integrated amount of irradiation with ultraviolet light of the human is reduced in comparison with a configuration in which both spaces are irradiated with ultraviolet light by the same integrated amount of irradiation, and it is less likely to reach the threshold limit value described above. Stated another way, an inactivation apparatus having further improved safety is achieved.

Furthermore, in the inactivation apparatus, the human detecting sensor may be provided to have the sensing region that moves according to the irradiation region changing mechanism driving by the driving unit.

Examples of the human detecting sensor include a sensor that receives and senses infrared light, and the like, and the sensor that receives and senses infrared light does not observe an amount of infrared light emitted from a human, but reacts to a movement of an object that emits infrared light, and performs sensing. In other words, in a specified fixed region, a human detecting sensor that is provided to sense the presence of a human fails to precisely sense a human that is stationary in a sensing region where infrared light is observed.

By employing the configuration described above, the irradiation region changing mechanism causes a sensing region of the human detecting sensor to move. Then, when viewed from the human detecting sensor, the stationary human appears to be relatively performing an operation in the sensing region. Accordingly, by employing the configuration described above, even in a state where a human is stationary in a space, the human detecting sensor can precisely sense the presence of the human.

The inactivation apparatus may include a direction data storage that stores direction data of a direction in which irradiation with the ultraviolet light is not performed.

It is conceivable that some operators, patients, or the like feel uncomfortable to be irradiated with ultraviolet light, or some operators, patients, or the like are to avoid being irradiated with ultraviolet light due to photodermatosis. It is also conceivable, for example, that articles that are to avoid being irradiated with ultraviolet light, such as photosensitive materials or chemicals that react to ultraviolet light, are present. In such cases, by storing a direction in which irradiation with ultraviolet light is not performed, inactivation can be appropriately performed in an environment.

The inactivation apparatus may include a direction data storage that stores direction data of a direction in which irradiation with the ultraviolet light is performed.

As described above, in some cases, more pathogens adhere to articles that are frequently touched by humans, such as desks or chairs, than ornaments or the like that are rarely touched by humans. In such a case, if the entire space is uniformly irradiated with ultraviolet light, there is a possibility that inactivation treatment will be insufficiently performed on the articles. In view of this, by employing the configuration described above, in a case where an irradiation target object that requires more intensive irradiation with ultraviolet light is present in a space or in other cases, inactivation treatment can be performed to intensively irradiate the irradiation target object with ultraviolet light.

Moreover, the inactivation apparatus may include:

a distance sensor that measures a distance of separation between the light source unit and an irradiation target object that is irradiated with the ultraviolet light according to the direction data; and
a second lighting controller that controls an emission intensity or a lighting time of the light source unit on the basis of a measurement result of the distance sensor.

In a case where a target that the light source will irradiate with ultraviolet light is sequentially changed, in some cases, a distance of separation between an article that is irradiated with ultraviolet light at a certain time and the light source unit is greater than a distance of separation between an article that has been previously irradiated with ultraviolet light and the light source unit.

In such cases, if the light source unit lights up all articles at the same emission intensity, in some cases, an article that is disposed in a position farther from the light source unit receives an insufficient amount of irradiation of ultraviolet light, and inactivation treatment fails to be sufficiently performed.

In view of this, by employing the configuration described above, in a case where an irradiation target object that is far from the light source unit is irradiated with ultraviolet light, for example, control can be performed to increase power to be supplied to the light source unit and improve an amount of irradiation of ultraviolet light to be applied to the irradiation target object, and inactivation treatment can be efficiently performed.

The inactivation apparatus may include

at least a human detection unit that senses whether a human is present in a space, and
when the human detection unit has sensed presence of the human in the space, the driving unit may drive the irradiation region changing mechanism to irradiate with a principal ray of the ultraviolet light, an outside of a sensing region at a time of human sensing of the human detection unit that has sensed the presence of the human.

In the inactivation apparatus, when the human detection unit has sensed the presence of the human in the space, the driving unit may drive the irradiation region changing mechanism to change the irradiation direction of the ultraviolet light in a state where the light source unit maintains irradiation of a first space is a space located at a height of less than 2 m from a floor in the space.

In the inactivation apparatus, the human detection unit may be provided to have the sensing region that moves according to the irradiation region changing mechanism driving by the driving unit.

In the inactivation apparatus, at a time of driving of the irradiation region changing mechanism, when the human detection unit has sensed a human absence region where the human is absent in the sensing region, the driving unit may drive the irradiation region changing mechanism to at least temporarily maintain the irradiation direction of the principal ray of the ultraviolet light emitted from the light source unit in the human absence region.

In the inactivation apparatus, when the human detection unit has sensed the presence of the human in the space, the driving unit may drive the irradiation region changing mechanism to cause the light source unit to at least temporarily irradiate a second space with the ultraviolet light, the second space is a space located at a height of 2 m or more from the floor.

In the inactivation apparatus, the human detection unit may be provided to have the sensing region that is fixed.

The inactivation apparatus may include

a plurality of the human detection units that is provided to have the sensing region that is fixed in the space, and
when any of the plurality of the human detection units has sensed the presence of the human, the driving unit may drive the irradiation region changing mechanism to change the irradiation direction of the principal ray of the ultraviolet light of the light source unit to the outside of the sensing region of the human detection unit that has sensed the presence of the human.

In the inactivation apparatus, when any of the plurality of the human detection units has sensed the presence of the human, and another of the plurality of the human detection units has detected a human absence region where the human is absent in the sensing region, the driving unit may drive the irradiation region changing mechanism to change the irradiation direction of the principal ray of the ultraviolet light emitted from the light source unit to an inside of the human absence region.

In the inactivation apparatus,

the driving unit may be able to drive the irradiation region changing mechanism to switch a direction in which the light source unit performs irradiation with the ultraviolet light between a first space and a second space, the first space is a space located at a height of less than 2 m from a floor, and the second space is a space located at a height of more than 2 m from the floor, and
when half or more of the plurality of the human detection units has sensed the presence of the human, the driving unit may drive the irradiation region changing mechanism to switch the irradiation direction of the ultraviolet light of the light source unit from the first space to the second space.

The human detection unit described here does not only include a human detecting sensor, but also includes a configuration that images a predetermined region by using a camera and analyzes a captured image to sense the presence of a human.

By employing the configuration described above, the inactivation apparatus can efficiently perform inactivation treatment in a space while avoiding irradiation of a human in the space with ultraviolet light having a high intensity.

An inactivation system for bacteria or viruses according to the present invention is a system for inactivating bacteria or viruses that are present in a space, the system including:

a light source unit that emits ultraviolet light having a peak wavelength within a wavelength range of 190 nm or more and less than 240 nm;
an irradiation region changing mechanism that changes an irradiation direction of the ultraviolet light of the light source unit;

a driving unit that drives the irradiation region changing mechanism to change, with time, the irradiation direction of the ultraviolet light of the light source unit;

a receiver that receives, from an outside, a wireless signal that has been predetermined for starting inactivation treatment, and outputs, to the driving unit, an operation start signal for starting a driving operation; and

a communication device that transmits, to the receiver, the wireless signal for starting the inactivation treatment.

The inactivation system may include

a pattern storage that stores data of an operation pattern of the irradiation region changing mechanism, and

the driving unit may drive the irradiation region changing mechanism on the basis of the data stored in the pattern storage.

The communication device described here is, for example, an operation panel that is installed in the space, a communication terminal that can perform data communication with an external device via wireless communication, such as a smartphone, a tablet, or a PC, or the like.

By employing the configuration described above, the inactivation apparatus can be operated from a position away from the inactivation apparatus or in a state where an operator is not present in the space. Therefore, an emission intensity or a direction of irradiation with ultraviolet light of the light source unit can be controlled without exposure of the operator to ultraviolet light emitted from the light source unit. Furthermore, in a space that is difficult to enter or exit from, for example, in a hospital ward where patients who suffer from an infectious disease or the like are in hospital, an emission intensity or a direction of irradiation with ultraviolet light of the light source unit can be controlled from the outside.

In the inactivation system,

when the receiver has received, from the communication device, a signal for refusing the ultraviolet light, the receiver may output a refusal sensing signal to the driving unit, and

when the driving unit has received the refusal sensing signal, the driving unit may drive the irradiation region changing mechanism to irradiate the ultraviolet light from the light source unit in a direction that is different from a direction in which the communication device is present.

Furthermore, the inactivation system may include

a first lighting controller that controls an emission intensity or a lighting time of the light source unit, and

when the receiver has received, from the communication device, a signal for refusing the ultraviolet light, the receiver may output a refusal sensing signal to the first lighting controller, and

when the first lighting controller has received the refusal sensing signal, the first lighting controller may control the light source unit to reduce light or be turned off.

As described above, it is conceivable that some operators, patients, or the like feel uncomfortable to be irradiated with ultraviolet light, or some operator, patients, or the like are to avoid being irradiated with ultraviolet light due to photodermatosis. It is also conceivable, for example, that articles that are to avoid being irradiated with ultraviolet light, such as photosensitive materials or chemicals that react to ultraviolet light, are present.

Accordingly, the system configuration described above enables a configuration that does not apply ultraviolet light to persons that do not desire to be irradiated with ultraviolet light, and an inactivation system that can conform to a wide range of users can be constructed.

EFFECT OF THE INVENTION

The present invention achieves an apparatus for inactivating bacteria or viruses and a system for inactivating bacteria or viruses that are capable of irradiating a wide range in a space with ultraviolet light that enables bacteria or viruses to be inactivated, and have improved safety.

BRIEF DESCRIPTION OF THE DRAWINGS

Fig. 1 is a diagram schematically illustrating an embodiment of an inactivation system.
Fig. 2 is a diagram illustrating a configuration of the inactivation apparatus of Fig. 1.
Fig. 3 is a block diagram schematically illustrating a configuration of a controller.
Fig. 4 is a diagram illustrating a state during an operation of the inactivation system illustrated in Fig. 1.
Fig. 5A is a timing chart relating to control performed on a light source unit by the controller.
Fig. 5B is a timing chart relating to control performed on the light source unit by the controller.
Fig. 5C is a timing chart relating to control performed on the light source unit by the controller.
Fig. 6 is a graph indicating a result of calculating a time change in the concentration of viruses floating in a room as a result of inactivation treatment.
Fig. 7 is a diagram schematically illustrating an embodiment of an inactivation system.

Fig. 8 is a diagram illustrating a configuration of the inactivation apparatus of Fig. 7.

Fig. 9 is a diagram schematically illustrating an embodiment of an inactivation system.

Fig. 10 is a diagram illustrating a configuration of the inactivation apparatus of Fig. 9.

Fig. 11 is a diagram schematically illustrating an embodiment of an inactivation system.

Fig. 12 is a diagram illustrating a configuration of the inactivation apparatus of Fig. 11.

Fig. 13 is a diagram illustrating a state during an operation of the inactivation system illustrated in Fig. 11.

Fig. 14 is a diagram schematically illustrating an embodiment of an inactivation system.

Fig. 15 is a block diagram schematically illustrating a configuration of a controller.

Fig. 16 is a diagram schematically illustrating an embodiment of an inactivation system.

Fig. 17 is a block diagram schematically illustrating a configuration of a controller.

Fig. 18 is a diagram schematically illustrating an embodiment of an inactivation system.

Fig. 19 is a diagram schematically illustrating an embodiment of an inactivation system.

Fig. 20 is a diagram schematically illustrating a state during an operation of the inactivation system illustrated in Fig. 19.

Fig. 21 is a graph indicating a characteristic of an average absorption coefficient in an ultraviolet light region of protein.

Fig. 22A is a graph indicating a time change in a survival rate after irradiation of Staphylococcus aureus with ultraviolet light by using a low-pressure mercury lamp.

Fig. 22B is a graph indicating a time change in a survival rate after irradiation of Staphylococcus aureus with ultraviolet light by using a KrCl excimer lamp.

Fig. 23 is a graph indicating an absorption spectrum of Escherichia coli (E. Coli).

MODE FOR CARRYING OUT THE INVENTION

An inactivation apparatus and an inactivation system according to the present invention will be described below with reference to the drawings. Note that each of the drawings described below is schematic illustration, and a dimensional ratio or the number of pieces in the drawings do not necessarily coincide with an actual dimensional ratio or the actual number of pieces.

[First embodiment]

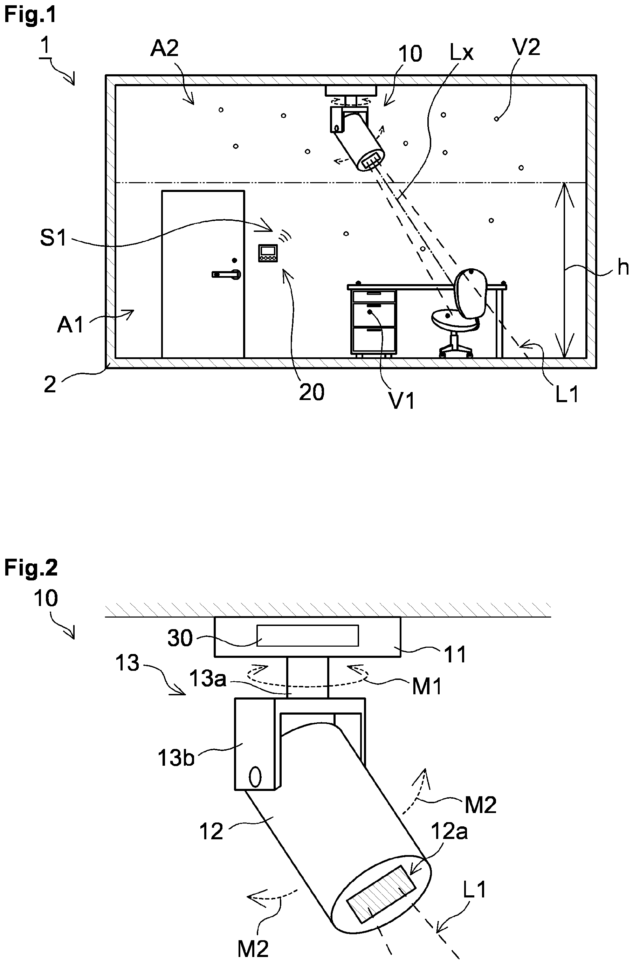

Fig. 1 is a diagram schematically illustrating a first embodiment of an inactivation system 1. As illustrated in Fig. 1, the inactivation system 1 includes an inactivation apparatus 10, and an operation panel 20 that corresponds to a communication device.

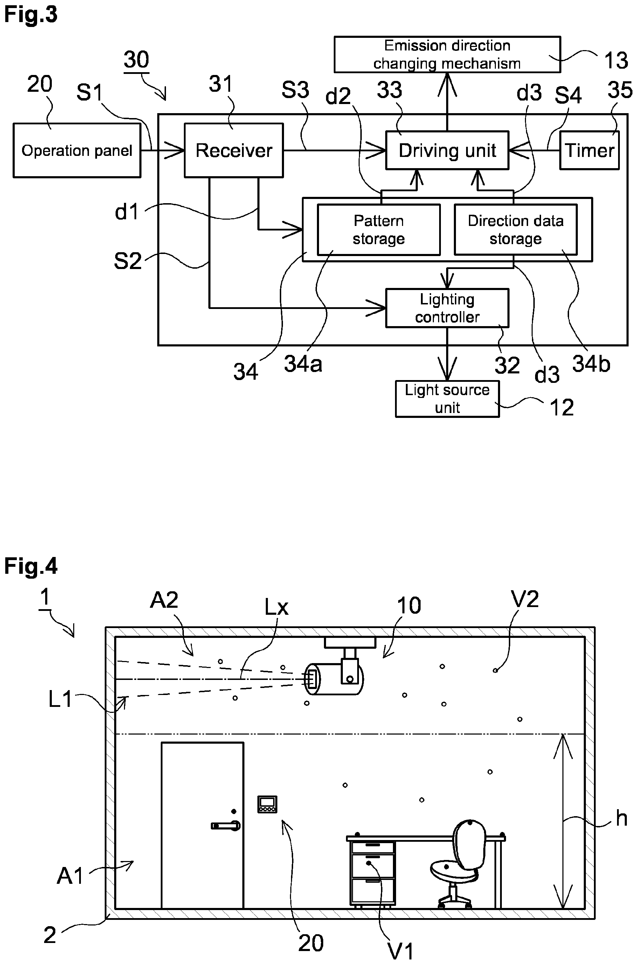

As illustrated in Fig. 1, in the inactivation system 1, the inactivation apparatus 10 is installed on a ceiling in a room 2. When the receiver 31 described later (see Fig. 3) has received, from the operation panel 20, a wireless signal S1 for starting an operation, the inactivation apparatus 10 starts to irradiate, with ultraviolet light L1, viruses V1 (illustrated as • in Fig. 1) that adhere to a desk, a chair, or the like in the room 2 or viruses V2 (illustrated as ∘ in Fig. 1) that adhere to aerosol or the like and are floating in the air. Then, the inactivation apparatus 10 performs an operation to automatically irradiate the entirety of the room 2 with ultraviolet light L1 on the basis of data stored in the pattern storage 34a described later (see Fig. 3).

Fig. 2 is a diagram illustrating a configuration of the inactivation apparatus 10 of Fig. 1. As illustrated in Fig. 2, the inactivation apparatus 10 includes a base 11 that is fixed to the ceiling, a light source unit 12 that emits ultraviolet light L1, an irradiation region changing mechanism 13 that couples the base 11 and the light source unit 12, and a controller 30.

The light source unit 12 includes an emission window 12a that emits ultraviolet light L1, as illustrated in Fig. 2. Furthermore, the light source unit 12 is coupled to the base 11 with the irradiation region changing mechanism 13 interposed therebetween.

In Fig. 1, the light source unit 12 is irradiating the chair disposed in a first space A1 with a principal ray Lx. Stated another way, Fig. 1 illustrates a state where the light source unit 12 is irradiating the first space A1 with ultraviolet light L1. Here, as illustrated in Fig. 1, the first space A1 is a region is a space located at a height h from the floor in the room 2, and the second space A2 is a region located above the first space A1.

Here, in the first embodiment, the height h from the floor has been set to be 2 m, but may be arbitrarily set. In a case where it is desired to make a distinction depending on whether a human is irradiated with ultraviolet light L1, it is preferable that the height h be 1.9 m or more and less than 2.1 m, and it is more preferable that the height h be 1.95 m or more and less than 2.05 m.

The light source unit 12 according to the first embodiment is configured to emit ultraviolet light L1 having a peak wavelength of 222 nm, and in order to reduce a light intensity in a wavelength range of 240 nm or more and less than 280 nm, the emission window 12a is provided with a not-illustrated optical filter. The optical filter provided in the emission window 12a of the light source unit 12 is constituted, for example, by a dielectric multilayer film. As the light source unit 12, for example, an excimer lamp in which krypton (Kr) and chlorine (Cl) have been sealed as light emission gas into a tube body can be employed. Note that in a case where a light intensity in a wavelength range of 240 nm or more and less than 280 nm of light emitted from the light source unit 12 is so low that the light intensity does not need to be reduced by the optical filter, the optical filter may be omitted.

The irradiation region changing mechanism 13 includes a first rotational movement mechanism 13a that is configured to rotationally move in the direction illustrated as arrow M1, and a second rotational movement mechanism 13b that is configured to cause the light source unit 12 to rotationally move in the direction of arrow M2, as illustrated in Fig. 2.

The first rotational movement mechanism 13a and the second rotational movement mechanism 13b are driven by a driving unit 33 (see Fig. 3) included in the controller 30 described later to move a direction in which the emission window 12a of the light source unit 12 is directed.

Fig. 3 is a block diagram schematically illustrating a configuration of the controller 30. As illustrated in Fig. 3, the controller 30 includes the receiver 31, a lighting controller 32 that corresponds to the first lighting controller, the driving unit 33, a storage 34, and a timer 35.

When the receiver 31 has received, from the operation panel 20, the wireless signal S1 that reports the start of an operation, the receiver 31 outputs a lighting signal S2 to the lighting controller 32, stands by for a time when the light source unit 12 is turned on, and then outputs, to the driving unit 33, an operation start signal S3 for starting a driving operation. In a case where the wireless signal S1 received from the operation panel 20 includes data d1 relating to an operation pattern, the receiver 31 stores the data d1 in the pattern storage 34a. whichever of an output of the lighting signal S2 and an output timing of the operation start signal S3 may come first, and the lighting signal S2 and the operation start signal S3 may be the same signal. Moreover, when the receiver 31 has received the wireless signal S1, the receiver 31 may only output the operation start signal S3 to the driving unit 33, and the light source unit 12 may be always on.

When the lighting controller 32 has received the lighting signal S2 as an input from the receiver 31, the lighting controller 32 starts to supply power to the light source unit 12 to put on the light source unit 12. Here, the lighting controller 32 is, for example, an electric circuit or the like in which output power is switched in response to the lighting signal S2.

When the driving unit 33 has received the operation start signal S3 as an input from the receiver 31, the driving unit 33 reads data d2 stored in advance of an operation pattern of the irradiation region changing mechanism 13, from the pattern storage 34a included in the storage 34. Then, the driving unit 33 drives the irradiation region changing mechanism 13 to operate according to the operation pattern based on the data d2.

Here, the driving unit 33 is, for example, an actuator or the like that drives the irradiation region changing mechanism 13 on the basis of the data d2, and includes a power source such as a motor, a control circuit, and mechanism parts. Furthermore, the storage 34 is, for example, a flash memory or the like that stores electronic data. Moreover, the entirety of the controller 30 may be constituted by a single CPU, microcontroller, or the like.

Fig. 4 is a diagram illustrating a state during an operation of the inactivation system 1 illustrated in Fig. 1. In Fig. 4, the light source unit 12 is irradiating a wall of the second space A2 with the principal ray Lx. Stated another way, Fig. 4 illustrates a state where the light source unit 12 is irradiating the second space A2 with ultraviolet light L1.

In the first embodiment, the pattern storage 34a stores the data d2 of an operation pattern in which the light source unit 12 alternately irradiates the first space A1 and the second space A2 with ultraviolet light L1 at predetermined time intervals. Stated another way, the driving unit 33 drives the irradiation region changing mechanism 13 to switch, at predetermined time intervals, a state where the light source unit 12 irradiates the first space A1 with the ultraviolet light L1, as illustrated in Fig. 1, and a state where the light source unit 12 irradiates the second space A2 with ultraviolet light L1, as illustrated in Fig. 4.

Furthermore, the operation pattern of the irradiation region changing mechanism 13 that is indicated by the data d2 is not limited to an operation pattern in which the driving unit 33 alternately switches an irradiation region between the first space A1 and the second space A2, but may be an operation pattern in which the driving unit 33 gradually moves a direction of irradiation with ultraviolet light L1 from a floor surface toward the ceiling, or another operation pattern.

The storage 34 according to the first embodiment includes, separately from the pattern storage 34a, a direction data storage 34b that stores data d3 relating to a position where a specified irradiation target object to be intensively irradiated with ultraviolet light L1 (for example, the chair illustrated in Fig. 1) is disposed.

When an operation based on the data d2 has been completed, the lighting controller 32 and the driving unit 33 read the data d3 from the direction data storage 34b. Then, the driving unit 33 drives the irradiation region changing mechanism 13 on the basis of the data d3 to direct the emission window 12a of the light source unit 12 in a direction in which the irradiation target object is located, and the lighting controller 32 supplies power to the light source unit 12 during a predetermined time period. Note that the irradiation target object may be intensively irradiated with ultraviolet light L1 before inactivation treatment is performed according to the operation pattern, or the irradiation target object may be intensively irradiated at a timing when the emission window 12a of the light source unit 12 is directed to the irradiation target object during inactivation treatment according to the operation pattern.

The timer 35 measures a time period of irradiation of the room 2 with ultraviolet light L1 from the start of an operation, and outputs, to the driving unit 33, a switching signal S4 that reports a timing of switching a direction in which the light source unit 12 applies ultraviolet light L1 between the first space A1 and the second space A2.

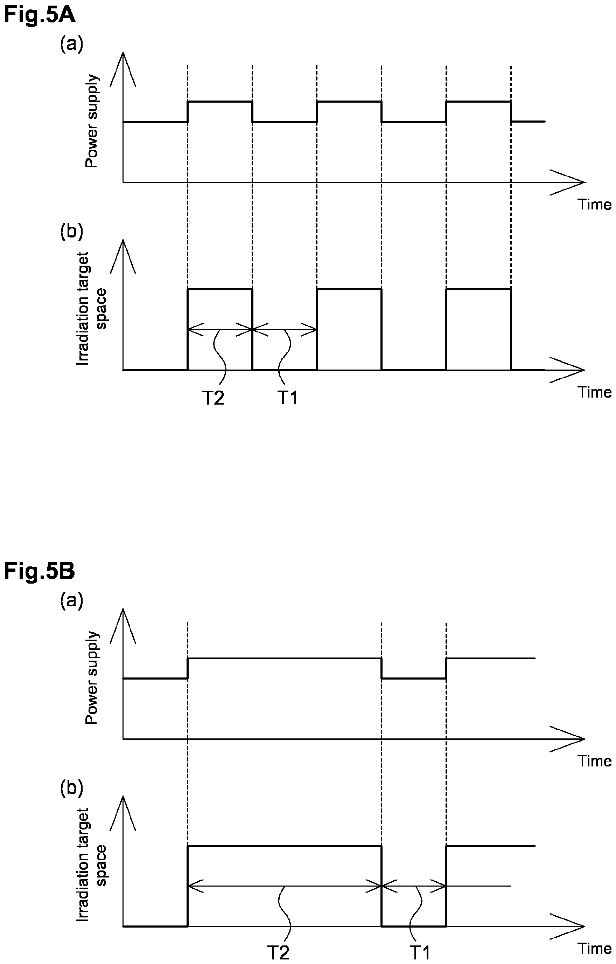

Fig. 5A is a timing chart relating to control performed on the light source unit 12 by the controller 30. In Fig. 5A, (a) is a timing chart of power that the lighting controller 32 supplies to the light source unit 12, and (b) is a timing chart of switching performed by the driving unit 33 of a space (A1 or A2) irradiated with ultraviolet light L1. In (b) of Fig. 5A, a region indicated as a high level indicates a state where the light source unit 12 is irradiating the second space A2 with ultraviolet light L1.

The lighting controller 32 according to the first embodiment performs control to supply more power to the light source unit 12 when the driving unit 33 controls the light source unit 12 to irradiate the second space A2 with ultraviolet light L1 than power at a time when the driving unit 33 controls the light source unit 12 to irradiate the first space A1 with ultraviolet light L1, as illustrated in Fig. 5A. Stated another way, an emission intensity of the light source unit 12 is higher during a period T2 during which the driving unit 33 controls the light source unit 12 to irradiate the second space A2 with ultraviolet light L1 than an emission intensity during a period T1 during which the driving unit 33 controls the light source unit 12 to irradiate the first space A1 with ultraviolet light L1.

Note that the emission intensity does not necessarily need to be changed depending on which space (A1 or A2) the light source unit 12 is irradiating with ultraviolet light L1. However, in a case where the first space A1 where a human comes and goes is irradiated with ultraviolet light L1, in order to reduce an amount of irradiation of the human with ultraviolet light L1, it is preferable that setting be performed to reduce the emission intensity or reduce a lighting time.

Fig. 5B is a timing chart relating to control performed on the light source unit 12 by the controller 30, and is a timing chart that is different from the timing chart of Fig. 5A. As illustrated in Fig. 5B, the controller 30 may perform control to reduce the emission intensity and reduce a time period during the period T2 during which the light source unit 12 irradiates the second space A2 with ultraviolet light L1 in comparison with the period T1 during which the light source unit 12 irradiates the first space A1 with ultraviolet light L1.

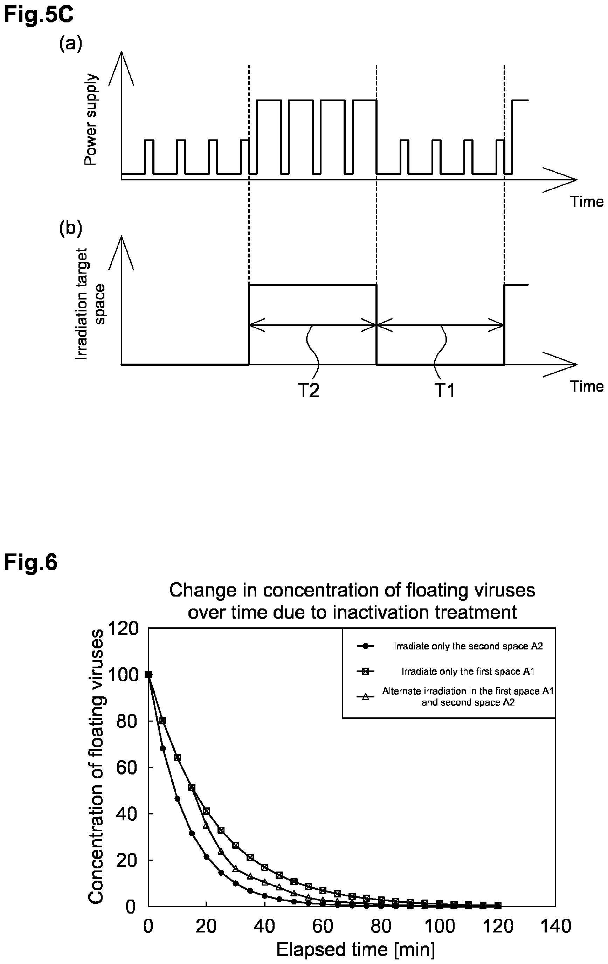

Furthermore, Fig. 5C is a timing chart relating to control performed on the light source unit 12 by the controller 30, and is a timing chart that is further different from the timing charts of Figs. 5A and 5b. As illustrated in Fig. 5C, the lighting controller 32 may control power to be supplied to the light source unit 12 to periodically turn off or reduce light. In all of Figs. 5A to 5C, the lighting controller 32 controls power to be supplied to the light source unit 12 in such a way that an amount of irradiation per unit time of ultraviolet light L1 that the light source unit 12 irradiates the first space A1 with is smaller than an amount of irradiation per unit time of ultraviolet light L1 that the light source unit 12 irradiates the second space A2 with.

Here, the content of calculation of a reduction in concentration of the viruses V1 that are floating in the room 2 in inactivation treatment is described in each of a case where an irradiation target region is alternately switched between the first space A1 and the second space A2 and a case where an irradiation direction is fixed to one irradiation target region.

In the calculation described here, it is assumed that inactivation treatment is performed by using a single inactivation apparatus 10 installed on the ceiling, as illustrated in Fig. 1, in the room 2 having a width of 6 m × 4 m × 2.5 m (width × depth × height). It is assumed that the first space A1 is a region is a space located at a height of less than 2 m from the floor, and the second space A2 is a region is a space located at a height of 2 m or more from the floor.

It is assumed that the inactivation apparatus 10 is installed in a center of the ceiling of the room 2, ultraviolet light L1 emitted from the light source unit 12 is conically emitted from the emission window 12a of the light source unit 12, the first space A1 is irradiated from the ceiling toward the floor by a distance of 2.5 m, and the second space A2 is irradiated from a center of the room 2 toward a wall on one side by a distance of 3 m. Note that, taking it into consideration that half of the ultraviolet light L1 applied to the second space A2 continues to be applied to the ceiling, it is assumed that an irradiation volume of ultraviolet light L1 applied to the second space A2 is half the volume of a conical shape.

It is assumed that the viruses V1 that are floating in the room 2 come and go between the first space A1 and the second space A2 twice per minute due to natural convection. Note that irradiation of the first space A1 with ultraviolet light L1 has very little to do with viruses coming and going between the first space A1 and the second space A2, and it is assumed that viruses coming and going do not affect the irradiation.

In the calculation, under the assumption that an initial bacteria concentration is $C_0$, a lighting time is T, a volume of the room 2 is R, an inactivation rate (a sterilization rate) of air is $\varepsilon$, and a total amount of air inactivated during a unit time is V, the concentration $C_T$ of the floating viruses V1 relative to the lighting time T was calculated according to Formulae (1) and (2) described below.

$$C_T = C_0 e^{(-\alpha T/R)} \quad (1)$$

$$\alpha = \varepsilon V \quad (2)$$

Fig. 6 is a graph indicating a result of calculating a time change in the concentration of the viruses V1 floating in the room 2 as a result of inactivation treatment, a vertical axis indicates the concentration of the floating viruses V1, and a horizontal axis indicates elapsed time. As illustrated in Fig. 6, a result indicates that the concentration of the floating viruses V1 most quickly decreases in a case where only the second space A2 is irradiated with ultraviolet light L1, and the concentration of the floating viruses V1 most slowly decreases in a case where only the first space A1 is irradiated with ultraviolet light L1. This is because due to circulation of the viruses V1 according to natural convection, an inactivation volume per unit time is greater in irradiation of the second space A2 with ultraviolet light L1 than irradiation of the first space A1 with ultraviolet light L1.

The result illustrated in Fig. 6 is merely a result for the viruses V1 floating in the air illustrated in Fig. 1 under the conditions described above. However, in the room 2, the viruses V2 that adhere to the floor, the desk, or the like are also present together with the floating viruses V1. As illustrated in Fig. 4, in a case where the second space A2 is being irradiated with ultraviolet light L1, the light source unit 12 does not irradiate the floor or the desk with ultraviolet light L1. Stated another way, in a case where only the second space A2 is being irradiated with ultraviolet light L1, many viruses V1 are reduced, but inactivation treatment is not performed at all on the viruses V2 that adhere to the floor or the desk.

In contrast, in a case where irradiation of the first space A1 with ultraviolet light L1 and irradiation of the second space A2 with ultraviolet light L1 are alternately switched, inactivation treatment is performed on the viruses V2 that adhere to the floor, the desk, or the like while the first space A1 is irradiated with ultraviolet light L1. In view of this point, from a viewpoint of efficiently inactivating viruses (V1, V2) that are present in the entirety of the room 2, a method for alternately irradiating the first space A1 and the second space A2 with ultraviolet light L1 can be considered most effective.

In view of the above, by employing the configuration described above, inactivation treatment can be performed on pathogens that are present in the room 2, in a state where a direction of irradiation with ultraviolet light L1 of the light source unit 12 automatically changes on the basis of the data d2 of the operation pattern of the irradiation region changing mechanism 13 that has been stored in advance in the pattern storage 34a.

Furthermore, inactivation treatment is performed by using ultraviolet light having a wavelength of 222 nm, which has almost no risk of affecting a human body. This achieves an inactivation system and an inactivation apparatus that have higher safety than a conventionally used inactivation apparatus using ultraviolet light having a wavelength of around 254 nm.

Note that in the inactivation apparatus 10 according to the first embodiment, the controller 30 includes the timer 35, and the light source unit 12 switches a direction of irradiation with ultraviolet light L1 between the first space A1 and the second space A2 at predetermined time intervals. However, the timer 35 may be omitted, and inactivation treatment may be simply performed in the order specified by the data d2 of the operation pattern.

Furthermore, in performing inactivation treatment in the room 2, the data d2 may indicate, for example, an operation pattern of the irradiation region changing mechanism 13 according to which inactivation treatment is sequentially performed from a side of a floor surface to the ceiling without making no distinction between the first space A1 and the second space A2 in the room 2.

Moreover, the control circuit or the mechanism parts may be omitted from the driving unit 33, and the irradiation region changing mechanism 13 may be simply configured to move an irradiation direction of ultraviolet light L1 of the light source unit 12 by only using an operation of the motor.

Moreover, the inactivation apparatus 10 may be configured to start inactivation treatment according to an operator pressing an operation start button provided in the inactivation apparatus 10 without using the wireless signal S1 transmitted from a communication device such as the operation panel 20, and inactivation treatment may be automatically started at a predetermined time.

Moreover, the data d3 stored in the direction data storage 34b is not limited to data relating to a position where a specified irradiation target object is disposed, and may be data relating to a position where an article that is not desired to be irradiated with ultraviolet light L1 is disposed or a position of a seat or a bed of a human who does not desire to be irradiated with ultraviolet light L1.

In a case where the data d3 is data relating to a position where an article that is not desired to be irradiated with ultraviolet light L1 is disposed or a position of a seat or a bed of a human that does not desire to be irradiated with ultraviolet light L1, for example, the driving unit 33 drives the irradiation region changing mechanism 13 to avoid irradiating the position where the article that is not desired to be irradiated with ultraviolet light L1 is disposed on the basis of the data d2.

Note that in a case where ultraviolet light L1 does not need to be intensively applied in a specified direction or in a case where ultraviolet light L1 does not need to be applied while avoiding a specified direction, the direction data storage 34b may be omitted.

The light source unit 12 included in the inactivation apparatus 10 according to the first embodiment is configured to emit ultraviolet light L1 having a wavelength of 222 nm, but it is sufficient if the wavelength of ultraviolet light L1 emitted by the light source unit 12 is 190 nm or more and less than 240 nm. Note that it is preferable that the wavelength of ultraviolet light L1 emitted by the light source unit 12 be 200 nm or more and 237 nm or less, it is more preferable that the wavelength be 200 nm or more and 235 nm or less, and it is particularly preferable that the wavelength be 200 nm or more and less than 230 nm.

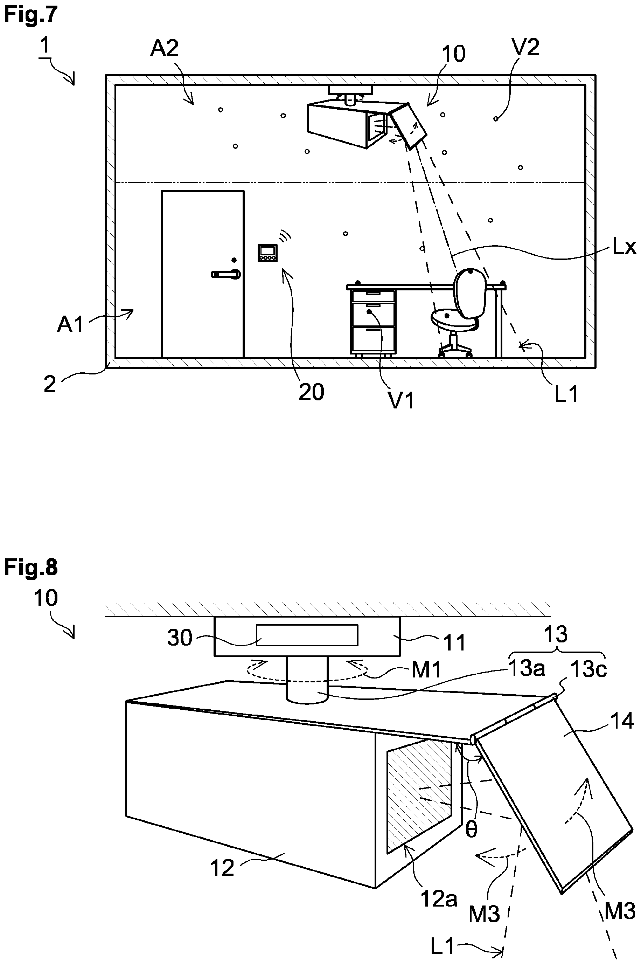

Fig. 7 is a diagram schematically illustrating a configuration of the inactivation system 1 that is different from the configuration of Fig. 1, and Fig. 8 is a diagram illustrating a configuration of the inactivation apparatus 10 of Fig. 7. As illustrated in Fig. 7, the inactivation apparatus 10 may include a mirror 14, and a third rotational movement mechanism 13c that rotationally moves the mirror 14 in the direction of arrow M3, instead of the second rotational movement mechanism 13b.

In the inactivation apparatus 10 illustrated in Fig. 8, the mirror 14 is rotationally moved in the direction of arrow M3 by the third rotational movement mechanism 13c, and therefore an angle θ of the mirror 14 relative to the emission window 12a changes, and a direction in which ultraviolet light L1 emitted from the emission window 12a of the light source unit 12 is reflected changes. In this case, a combination of the first rotational movement mechanism 13a and the third rotational movement mechanism 13c constitutes the irradiation region changing mechanism 13.

[Second embodiment]

A configuration of a second embodiment of the inactivation system 1 and the inactivation apparatus 10 according to the present invention is described focusing on a difference from the first embodiment.

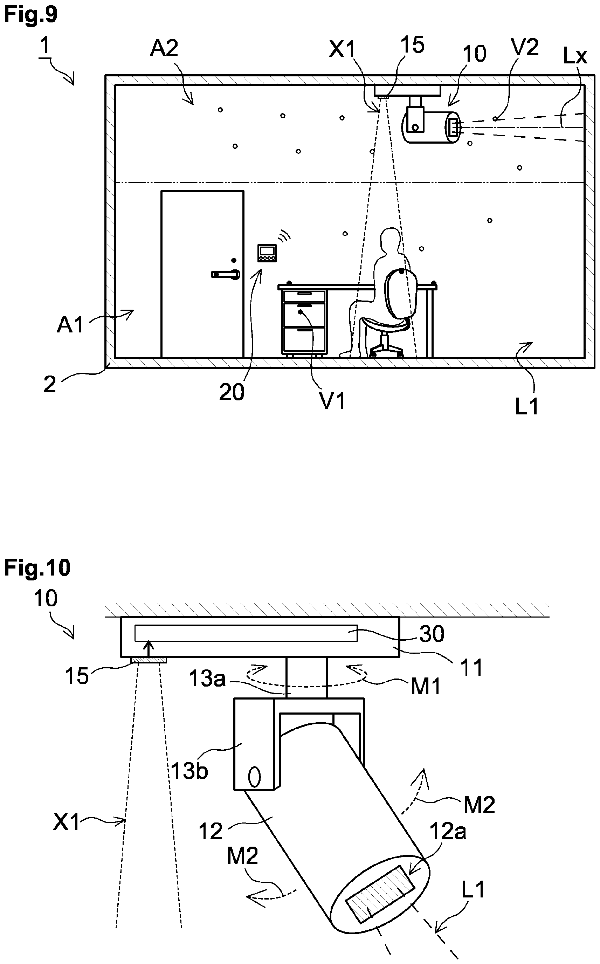

Fig. 9 is a diagram schematically illustrating the second embodiment of the inactivation system 1, and Fig. 10 is a diagram illustrating a configuration of the inactivation apparatus 10 of Fig. 9. As illustrated in Fig. 10, the inactivation apparatus 10 according to the second embodiment includes a first human detecting sensor 15 provided on the base 11 in such a way that a specified region in the room 2 is determined as a sensing region X1.

The first human detecting sensor 15 senses whether a human is present in the sensing region X1 that has been set within a specified range in the room 2. Note that in the second embodiment, the first human detecting sensor 15 is a sensor that senses infrared light emitted from a human in the sensing region X1 to sense the presence of the human. The first human detecting sensor 15 is not limited to the sensor that senses infrared light, and, for example, an ultrasonic sensor or the like can also be employed.

In the second embodiment, when the first human detecting sensor 15 has sensed the presence of a human, the driving unit 33 drives the irradiation region changing mechanism 13 to cause the light source unit 12 to irradiate the second space A2 with ultraviolet light L1. Then, when the first human detecting sensor 15 has sensed the absence of a human in the space, the driving unit 33 drives the irradiation region changing mechanism 13 to cause the light source unit 12 to irradiate the first space A1 with ultraviolet light L1.

The configuration described above enables a reduction in an amount of irradiation of ultraviolet light L1 applied to a human.

[Third embodiment]

A configuration of a third embodiment of the inactivation system 1 and the inactivation apparatus 10 according to the present invention is described focusing on a difference from the first embodiment and the second embodiment.

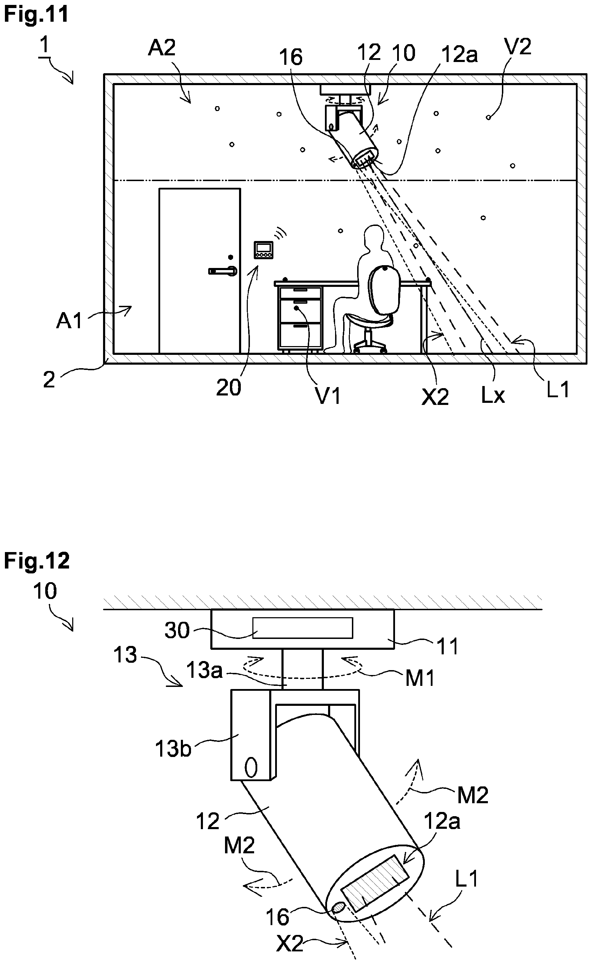

Fig. 11 is a diagram schematically illustrating the third embodiment of the inactivation system 1, and Fig. 12 is a diagram illustrating a configuration of the inactivation apparatus 10 of Fig. 11. As illustrated in Fig. 12, the inactivation apparatus 10 according to the third embodiment includes a second human detecting sensor 16 on the same face as a face on which the emission window 12a of the light source unit 12 is provided.

The second human detecting sensor 16 has a sensing region X2 that is moved by the irradiation region changing mechanism 13 in the room 2 in the same manner as a manner of ultraviolet light L1 emitted from the emission window 12a of the light source unit 12. Note that in the third embodiment, the second human detecting sensor 16 is a sensor that senses infrared light emitted from a human in the sensing region X2 to sense the presence of the human. The second human detecting sensor 16 is not limited to the sensor that senses infrared light, and, for example, an ultrasonic sensor or the like can also be employed.

In the third embodiment, when the second human detecting sensor 16 has sensed the presence of a human, the driving unit 33 drives the irradiation region changing mechanism 13 to cause the light source unit 12 to irradiate the second space A2 with ultraviolet light L1. Then, when the second human detecting sensor 16 has sensed the absence of a human in the space, the driving unit 33 drives the irradiation region changing mechanism 13 to cause the light source unit 12 to irradiate the first space A1 with ultraviolet light L1.

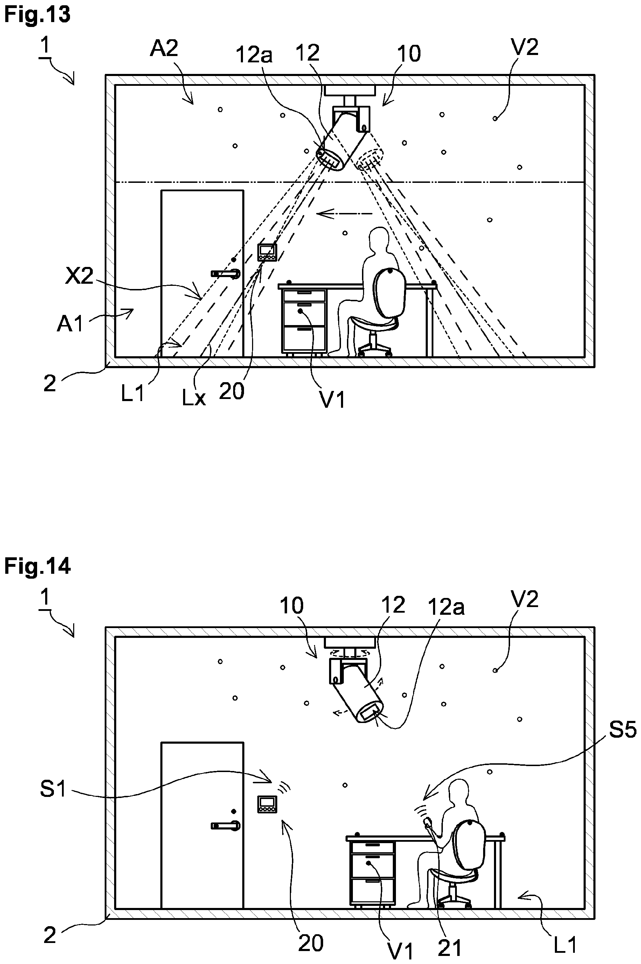

Fig. 13 is a diagram illustrating a state during an operation of the inactivation system 1 of Fig. 11. By employing the configuration described above, as illustrated in Fig. 12, the sensing region X2 of the second human detecting sensor 16 moves according to the light source unit 12 in which an emission direction of the ultraviolet light L1 is changed by the irradiation region changing mechanism 13. When the sensing region X2 of the second human detecting sensor 16 is moving to include a stationary human in the room 2, it appears that the human is relatively moving in the sensing region, when viewed from the second human detecting sensor 16.

Accordingly, by employing the configuration described above, even in a state where a human is stationary in the room 2, the second human detecting sensor 16 can sense the presence of the human in the room 2, and an amount of irradiation of ultraviolet light L1 applied to the human can be reduced.

[Fourth embodiment]

A configuration of a fourth embodiment of the inactivation system 1 and the inactivation apparatus 10 according to the present invention is described focusing on a difference from the first embodiment, the second embodiment, and the third embodiment.

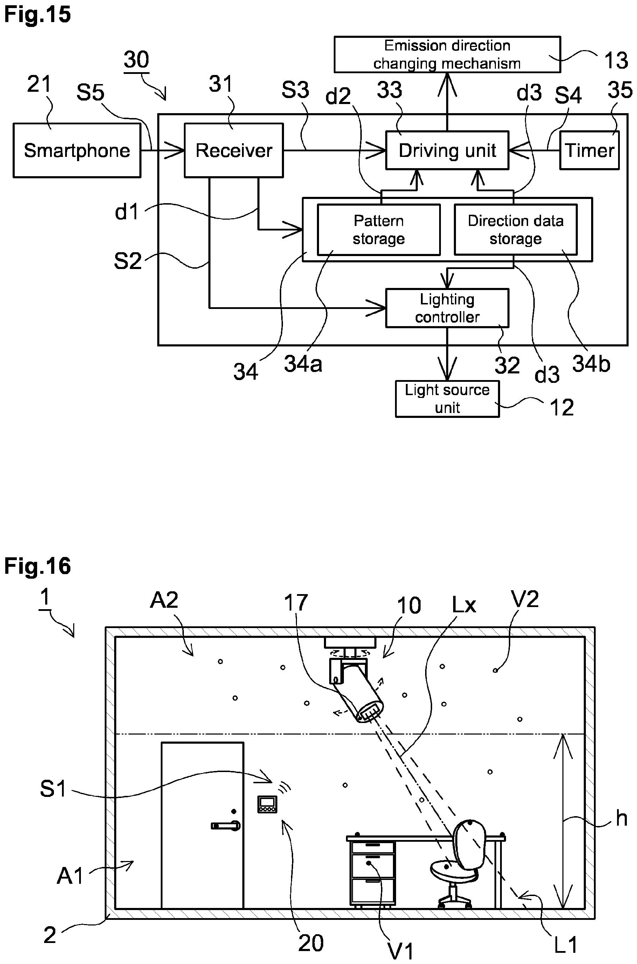

Fig. 14 is a diagram schematically illustrating the fourth embodiment of the inactivation system 1, and Fig. 15 is a block diagram schematically illustrating a configuration of the controller 30. In the controller 30 of the inactivation apparatus 10 according to the fourth embodiment, when the receiver 31 has received a refusal signal S5 transmitted from a smartphone 21, the receiver 31 outputs a not-illustrated refusal sensing signal to the lighting controller 32, as illustrated in Figs. 14 and 15. When the lighting controller 32 has received the refusal sensing signal as an input, the lighting controller 32 temporarily stops supplying power to the light source unit 12.

When the lighting controller 32 has received the refusal sensing signal as an input, the controller 30 of the inactivation apparatus 10 may reduce power to be supplied to the light source unit 12 instead of stopping the power. Furthermore, when the receiver 31 has received the refusal signal S5, the receiver 31 outputs the refusal sensing signal to the driving unit 33. When the driving unit 33 has received the refusal sensing signal as an input, the driving unit 33 may drive the irradiation region changing mechanism 13 to direct the emission window 12a of the light source unit 12 in a direction that is different from a direction in which the refusal signal S5 has been output.

By employing the configuration described above, the inactivation system 1 can be configured to not irradiate, with ultraviolet light L1 for inactivation treatment, humans that do not desire to be irradiated with ultraviolet light, such as humans who are concerned about damage to skin or humans who easily become sick.

The configuration illustrated in Fig. 14 indicates a single human who outputs the refusal signal S5 in the room 2. However, a plurality of humans may be present in the room 2, and the inactivation apparatus 10 may be able to simultaneously receive the refusal signal S5 from a plurality of smartphones 21.

[Fifth embodiment]

A configuration of a fifth embodiment of the inactivation system 1 and the inactivation apparatus 10 according to the present invention is described focusing on a difference from the first embodiment to the fourth embodiment.

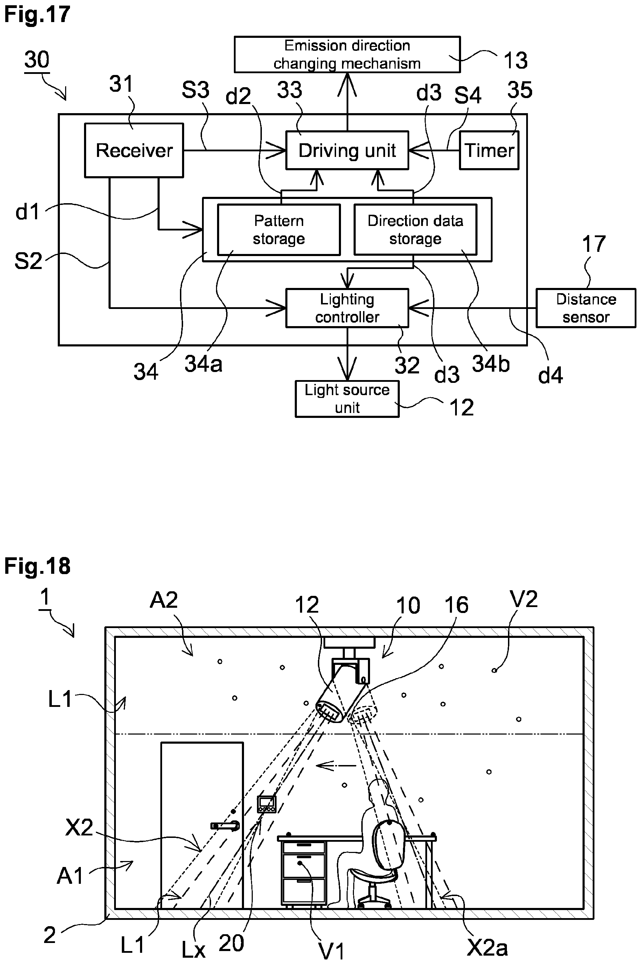

Fig. 16 is a diagram schematically illustrating the fifth embodiment of the inactivation system 1, and Fig. 17 is a block diagram schematically illustrating a configuration of the controller 30. As illustrated in Figs. 16 and 17, the inactivation apparatus 10 according to the fifth embodiment includes a distance sensor 17.

As illustrated in Fig. 17, the distance sensor 17 measures a distance of separation between the light source unit 12 and an irradiation target object, and outputs measurement data d4 including information indicating a measurement result to the lighting controller 32 of the controller 30. The lighting controller 32 that has received the measurement data d4 as an input performs control to increase power to be supplied to the light source unit 12 as the measurement data d4, that is, a distance of separation between the light source unit 12 and the irradiation target object increases. Note that the lighting controller 32 described here corresponds to the second lighting controller.

By employing the configuration described above, an amount of irradiation of ultraviolet light L1 can be improved for an irradiation target object that is far from the light source unit 12, and inactivation treatment can be promoted.

[Sixth embodiment]

A configuration of a sixth embodiment of the inactivation system 1 and the inactivation apparatus 10 according to the present invention is described focusing on a difference from the first embodiment to the fifth embodiment.

Fig. 18 is a diagram schematically illustrating the sixth embodiment of the inactivation system 1. A configuration of the sixth embodiment of the inactivation system 1 is the same as the configuration of the third embodiment illustrated in Fig. 12, and the sensing region X2 of the second human detecting sensor 16 that corresponds to the human detection unit moves according to the light source unit 12 in which an emission direction of ultraviolet light L1 is changed by the irradiation region changing mechanism 13.

When an operation to start an operation has been performed or when a predetermined time that has been set to start an operation has come, the sixth embodiment of the inactivation system 1 performs inactivation treatment in the room 2 while the irradiation region changing mechanism 13 changes a direction in which the light source unit 12 emits ultraviolet light L1. As illustrated in Fig. 18, when the second human detecting sensor 16 has sensed the presence of a human during a change in a direction of emission of ultraviolet light L1, the irradiation region changing mechanism 13 changes a direction in which the light source unit 12 emits ultraviolet light L1 in such a way that the principal ray Lx is applied to an outside of a sensing region X2a at a time when the second human detecting sensor 16 has sensed the presence of the human in the first space A1.

By employing the configuration described above, the inactivation system 1 can sequentially advance inactivation treatment in the room 2 while avoiding irradiation of a human in the room 2 with ultraviolet light L1.

Note that in the sixth embodiment, when the second human detecting sensor 16 has sensed the presence of a human, the irradiation region changing mechanism 13 changes an emission direction of the light source unit 12 in such a way that the principal ray Lx of the ultraviolet light L1 is applied to an outside of the sensing region X2a in the first space A1, but the irradiation region changing mechanism 13 may change the emission direction of the light source unit 12 in such a way that the principal ray Lx of the ultraviolet light L1 is applied to an inside of the second space A2.

By performing the operation described above, the principal ray Lx of ultraviolet light L1 is applied to an inside of the second space A2 in a case where the presence of a human has been sensed. This can reliably avoid irradiating a human with ultraviolet light L1 having a high intensity. Such control is effective, for example, in a case where there are many humans in the room 2, and a direction in which ultraviolet light L1 can be applied is narrow in the first space A1.

Furthermore, in the sixth embodiment, in a case where the second human detecting sensor 16 has sensed the presence of a human, an emission direction of the principal ray Lx of ultraviolet light L1 has been changed to a region outside the sensing region X2a, and then the second human detecting sensor 16 has detected a human absence region where no humans are sensed, irradiation with the principal ray Lx of ultraviolet light L1 may be maintained in the human absence region during a predetermined time period. In order to sufficiently perform inactivation treatment, it is preferable that the predetermined time period be 30 seconds or more, and it is more preferable that the predetermined time period be 60 seconds or more.

Moreover, in the sixth embodiment, the second human detecting sensor 16 is included as the human detection unit. However, the human detection unit is not limited to the human detecting sensor, and, for example, a camera that images a predetermined region in the room 2, and an image analyzer that analyzes an image captured by the camera and determines whether a human is present may be included.

Note that a sensing region in a case where the inactivation system 1 includes a camera as the human detection unit may be an imaging range of the camera in the room 2, or may be a specified range of an imaged region. In a case where the sensing region of the camera is a specified range of the imaged region, for example, the image analyzer analyzes an image captured by the camera to determine whether a human is present in the specified region.

[Seventh embodiment]

A configuration of a seventh embodiment of the inactivation system 1 and the inactivation apparatus 10 according to the present invention is described focusing on a difference from the first embodiment to the sixth embodiment.

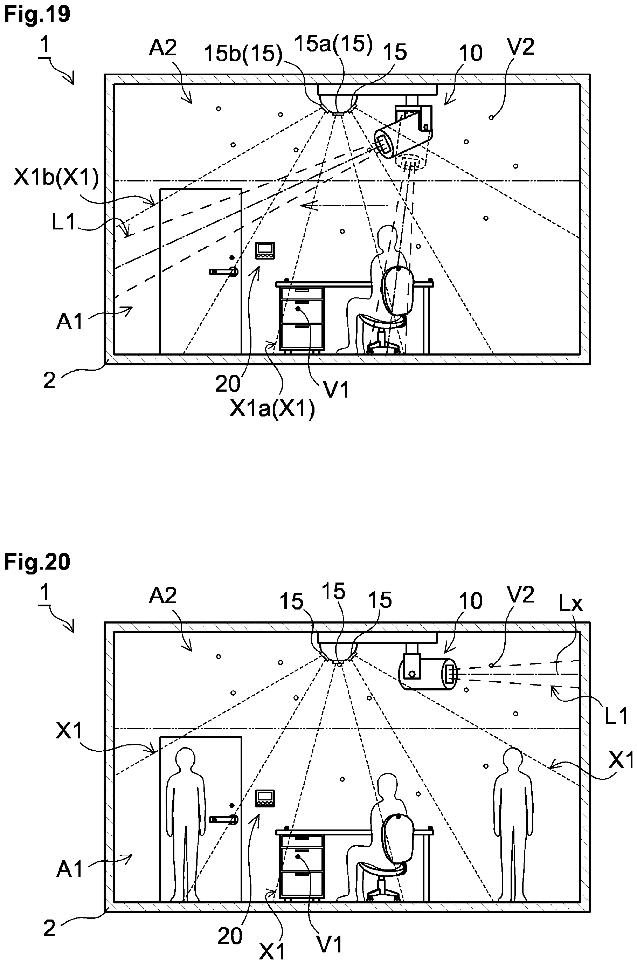

Fig. 19 is a diagram schematically illustrating the seventh embodiment of the inactivation system 1. As illustrated in Fig. 19, the seventh embodiment of the inactivation system 1 includes a plurality of first human detecting sensors 15 that corresponds to the human detection unit that has a fixed sensing region X1.

The plurality of first human detecting sensors 15 according to the seventh embodiment has been set in such a way that respective sensing regions X1 do not overlap each other, as illustrated in Fig. 19. However, the respective sensing regions X1 may be set in such a way that the respective sensing regions X1 partially overlap each other.

In the seventh embodiment of the inactivation system 1, when any of the plurality of first human detecting sensors 15 has sensed the presence of a human, the irradiation region changing mechanism 13 changes an emission direction of ultraviolet light L1 of the light source unit 12 in such a way that the principal ray Lx of ultraviolet light L1 is applied toward the sensing region X1 of the first human detecting sensor 15 that has sensed the human absence region where no humans are present.

An example of a specific operation is described with reference to Fig. 19. It is assumed that ultraviolet light L1 emitted from the light source unit 12 is applied toward an inside of the sensing region X1a of a first human detecting sensor 15a, while an inactivation operation of the inactivation system 1 is being performed. When a human has moved into the sensing region X1a, the irradiation region changing mechanism 13 changes an emission direction of ultraviolet light L1 of the light source unit 12 in such a way that the principal ray Lx of the ultraviolet light L1 is applied to an inside of a sensing region X1b (the human absence region) of a first human detecting sensor 15b that has not sensed a human, as illustrated in Fig. 19.

By employing the configuration described above, the inactivation system 1 can sequentially advance inactivation treatment in the room 2 while avoiding irradiation of a human in the room 2 with ultraviolet light L1.

Fig. 20 is a diagram schematically illustrating a state during an operation of the inactivation system 1 illustrated in Fig. 19. In the seventh embodiment of the inactivation system 1, as illustrated in Fig. 20, when all of the first human detecting sensors 15 have sensed the presence of a human, the irradiation region changing mechanism 13 changes an irradiation direction of ultraviolet light L1 of the light source unit 12 in such a way that the second space A2 is irradiated with ultraviolet light L1.

Note that in the seventh embodiment, in a case where all of the first human detecting sensors 15 have sensed the presence of a human, the second space A2 is irradiated with ultraviolet light L1. However, in a case where some of the first human detecting sensors 15 have sensed the presence of a human, the second space A2 may be irradiated with ultraviolet light L1. In a case where there are many humans in the room 2, from a viewpoint of avoiding irradiation of a human as much as possible, it is preferable that the second space A2 be irradiated with ultraviolet light L1 in a case where at least half of the first human detecting sensors 15 included in the inactivation system 1 have sensed a human.

In the seventh embodiment of the inactivation system 1, in a case where any of the first human detecting sensors 15 has sensed the presence of a human, the principal ray Lx of the ultraviolet light L1 is applied to the human absence region. However, the principal ray Lx of the ultraviolet light L1 may be applied to a region that is located outside a sensing region X1 of the first human detecting sensor 15 that has sensed the human and is different from sensing regions X1 of any other first human detecting sensors 15.

Furthermore, the seventh embodiment of the inactivation system 1 includes a plurality of first human detecting sensors 15, but a single first human detecting sensor 15 may be mounted. For example, in a case where the room 2 is not so wide, a case where a human goes in and out with low frequency, or another case, a single first human detecting sensor 15 may be mounted, and when the first human detecting sensor 15 has sensed the presence of a human, the principal ray Lx of ultraviolet light L1 may be applied to an outside of the sensing region X1 of the first human detecting sensor 15.

Furthermore, the inactivation system 1 according to the seventh embodiment may include a human detection unit other than a human detecting sensor, and for example, the inactivation system 1 may include a human detection unit constituted by a camera and an image analyzer similarly to the sixth embodiment.

Moreover, in any of the embodiments described above including the seventh embodiment, the human detection unit constituted by the camera and the image analyzer may be included additionally, together with the human detecting sensor, or instead of the human detecting sensor, and a human detection unit that determines whether a human is present by using a pressure sensor, a proximity sensor, or the like may be included.

DESCRIPTION OF REFERENCE SIGNS

| | |
|---|---|
| 1 | Inactivation system |
| 2 | Room |
| 10 | Inactivation apparatus |
| 11 | Base |
| 12 | Light source unit |
| 12a | Emission window |
| 13 | Irradiation region changing mechanism |
| 13a | First rotational movement mechanism |
| 13b | Second rotational movement mechanism |
| 13c | Third rotational movement mechanism |
| 14 | Mirror |
| 15, 15a, 15b | First human detecting sensor |
| 16 | Second human detecting sensor |
| 17 | Distance sensor |
| 20 | Operation panel |
| 21 | Smartphone |
| 30 | Controller |
| 31 | Receiver |
| 32 | Lighting controller |
| 33 | Driving unit |
| 34 | Storage |
| 34a | Pattern storage |
| 34b | Direction data storage |
| 35 | Timer |
| A1 | First space |
| A2 | Second space |
| L1 | Ultraviolet light |
| Lx | Principal ray |
| T1, T2 | Period |
| V1, V2 | Virus |
| X1, X1a, X1b, X2 | Sensing region |

**Claims**

1. An apparatus for inactivating bacteria or viruses in a space, the apparatus comprising:

a light source unit that emits ultraviolet light having a peak wavelength within a wavelength range of 190 nm or more and less than 240 nm;
an irradiation region changing mechanism that changes an irradiation direction of the ultraviolet light of the light source unit; and
a driving unit that drives the irradiation region changing mechanism to change the irradiation direction of the ultraviolet light of the light source unit.

2. The apparatus for inactivating the bacteria or the viruses according to claim 1, wherein the light source unit includes an optical filter configured to reduce a light intensity at least in a wavelength range of 240 nm or more and less than 280 nm.

3. The apparatus for inactivating the bacteria or the viruses according to claim 1, the apparatus further comprising a pattern storage that stores data of an operation pattern of the irradiation region changing mechanism, wherein the driving unit drives the irradiation region changing mechanism on a basis of the data stored in the pattern storage.

4. The apparatus for inactivating the bacteria or the viruses according to claim 1, wherein the driving unit drives the irradiation region changing mechanism to switch a direction of irradiation with the ultraviolet light of the light source unit between a first space and a second space in the space, the first space is a space located at a height of less than 2 m from the floor, and the second space is a space located at a height of more than 2 m from the floor.

5. The apparatus for inactivating the bacteria or the viruses according to claim 4, further comprising a first lighting controller that controls an emission intensity or a lighting time of the light source unit, wherein the first lighting controller controls the light source unit to cause an amount of irradiation per unit time of the first space with the ultraviolet light to be smaller than the amount of irradiation per the unit time of the second space with the ultraviolet light.

6. The apparatus for inactivating the bacteria or the viruses according to claim 5, wherein the first lighting controller controls the light source unit to cause the emission intensity of the light source unit at a time of irradiation of the first space with the ultraviolet light to be lower than the emission intensity of the light source unit at a time of irradiation of the second space with the ultraviolet light.

7. The apparatus for inactivating the bacteria or the viruses according to claim 4, further comprising a timer that measures an irradiation time of the ultraviolet light, wherein the driving unit drives the irradiation region changing mechanism to switch the irradiation of the first space with the ultraviolet light and the irradiation of the second space with the ultraviolet light on a basis of a time measured by the timer.

8. The apparatus for inactivating the bacteria or the viruses according to claim 7, wherein the driving unit drives the irradiation region changing mechanism to cause a time during which the light source unit will irradiate the second space with the ultraviolet light to be longer than a time during which the light source unit has irradiated the first space with the ultraviolet light immediately before.

9. The apparatus for inactivating the bacteria or the viruses according to claim 4, further comprising a human detecting sensor that senses whether a human is present in the space, wherein

when the human detecting sensor has sensed presence of the human in the space, the driving unit drives the irradiation region changing mechanism to cause the light source unit to irradiate the second space with the ultraviolet light, and when the human detecting sensor has sensed absence of the human in the space, the driving unit drives the irradiation region changing mechanism to cause the light source unit to irradiate the first space with the ultraviolet light.

10. The apparatus for inactivating the bacteria or the viruses according to claim 9, wherein the human detecting sensor is provided to have a sensing region that is fixed.

11. The apparatus for inactivating the bacteria or the viruses according to claim 9, wherein the human detecting sensor is provided to have a sensing region that moves according to the irradiation region changing mechanism driving by the driving unit.

12. The apparatus for inactivating the bacteria or the viruses according to any one of claims 1 to 11, further comprising a direction data storage that stores direction data of a direction in which irradiation with the ultraviolet light is not performed.

13. The apparatus for inactivating the bacteria or the viruses according to any one of claims 1 to 11, further comprising a direction data storage that stores direction data of a direction in which irradiation with the ultraviolet light is performed.

14. The apparatus for inactivating the bacteria or the viruses according to claim 13, further comprising:

a distance sensor that measures a distance of separation between the light source unit and an irradiation target object that is irradiated with the ultraviolet light according to the direction data; and
a second lighting controller that controls an emission intensity or a lighting time of the light source unit on a basis of a measurement result of the distance sensor.

15. The apparatus for inactivating the bacteria or the viruses according to claim 1, further comprising at least a human detection unit that senses whether a human is present in the space, wherein when the human detection unit has sensed presence of the human in the space, the driving unit drives the irradiation region changing mechanism to irradiate with a principal ray of the ultraviolet light, an outside of a sensing region at a time of human sensing of the human detection unit that has sensed the presence of the human.

16. The apparatus for inactivating the bacteria or the viruses according to claim 15, wherein when the human detection unit has sensed the presence of the human in the space, the driving unit drives the irradiation region changing mechanism to change the irradiation direction of the ultraviolet light in a state where the light source unit maintains irradiation of a first space is a space located at a height of less than 2 m from a floor in the space.

17. The apparatus for inactivating the bacteria or the viruses according to claim 15, wherein the human detection unit is provided to have the sensing region that moves according to the irradiation region changing mechanism driving by the driving unit.

18. The apparatus for inactivating the bacteria or the viruses according to claim 17, wherein at a time of driving of the irradiation region changing mechanism, when the human detection unit has sensed a human absence region where the human is absent in the sensing region, the driving unit drives the irradiation region changing mechanism to at least temporarily maintain the irradiation direction of the principal ray of the ultraviolet light emitted from the light source unit in the human absence region.

19. The apparatus for inactivating the bacteria or the viruses according to claim 17, wherein when the human detection unit has sensed the presence of the human in the space, the driving unit drives the irradiation region changing mechanism to cause the light source unit to at least temporarily irradiate a second space with the ultraviolet light, the second space is a space located at a height of 2 m or more from the floor.

20. The apparatus for inactivating the bacteria or the viruses according to claim 15, wherein the human detection unit is provided to have the sensing region that is fixed.

21. The apparatus for inactivating the bacteria or the viruses according to claim 20, further comprising a plurality of the human detection units that is provided to have the sensing region that is fixed in the space, wherein when any of the plurality of the human detection units has sensed the presence of the human, the driving unit drives the irradiation region changing mechanism to change the irradiation direction of the principal ray of the ultraviolet light of the light source unit to the outside of the sensing region of the human detection unit that has sensed the presence of the human.

22. The apparatus for inactivating the bacteria or the viruses according to claim 21, wherein when any of the plurality of the human detection units has sensed the presence of the human, and another of the plurality of the human detection units has detected a human absence region where the human is absent in the sensing region, the driving unit drives the irradiation region changing mechanism to change the irradiation direction of the principal ray of the ultraviolet light emitted from the light source unit to an inside of the human absence region.

23. The apparatus for inactivating the bacteria or the viruses according to claim 21 or 22, wherein

the driving unit is configured to be able to drive the irradiation region changing mechanism to switch a direction where the light source unit performs irradiation with the ultraviolet light between a first space and a second space, the first space is a space located at a height of less than 2 m from a floor, and the second space is a space located at a height of more than 2 m from the floor, and
when half or more of the plurality of the human detection units has sensed the presence of the human, the driving unit drives the irradiation region changing mechanism to switch the irradiation direction of the ultraviolet light of the light source unit from the first space to the second space.

24. A system for inactivating bacteria or viruses that are present in a space, the system comprising:

a light source unit that emits ultraviolet light having a peak wavelength within a wavelength range of 190 nm or more and less than 240 nm;
an irradiation region changing mechanism that changes an irradiation direction of the ultraviolet light of the light source unit;
a driving unit that drives the irradiation region changing mechanism to change, with time, the irradiation direction of the ultraviolet light of the light source unit;
a receiver that receives, from an outside, a wireless signal that has been predetermined for starting inactivation treatment, and outputs, to the driving unit, an operation start signal for starting a driving operation; and
a communication device that transmits, to the receiver, the wireless signal for starting the inactivation treatment.

25. The system for inactivating the bacteria or the viruses according to claim 24, the system further comprising a pattern storage that stores data of an operation pattern of the irradiation region changing mechanism, wherein the driving unit drives the irradiation region changing mechanism on a basis of the data stored in the pattern storage.

26. The system for inactivating the bacteria or the viruses according to claim 24 or 25, wherein

when the receiver has sensed reception from the communication device of a signal for refusing the ultraviolet light, the receiver outputs a refusal sensing signal to the driving unit, and
when the driving unit has received the refusal sensing signal, the driving unit drives the irradiation region changing mechanism to irradiate the ultraviolet light from the light source unit in a direction that is different from a direction in which the communication device is present.

27. The system for inactivating the bacteria or the viruses according to claim 24 or 25, further comprising a first lighting controller that controls an emission intensity or a lighting time of the light source unit, wherein

when the receiver has received, from the communication device, a signal for refusing the ultraviolet light, the receiver outputs a refusal sensing signal to the first lighting controller, and when the first lighting controller has received the refusal sensing signal, the first lighting controller controls the light source unit to reduce light or be turned off.

**Fig.1**

**Fig.2**

# Fig.3

# Fig.4

**Fig.5A**

(a)

Power supply

Time

(b)

Irradiation target space

T2    T1

Time

**Fig.5B**

(a)

Power supply

Time

(b)

Irradiation target space

T2    T1

Time

# Fig.5C

# Fig.6

**Fig.7**

**Fig.8**

**Fig.9**

**Fig.10**

**Fig.11**

**Fig.12**

**Fig.13**

**Fig.14**

**Fig.15**

**Fig.16**

**Fig.17**

**Fig.18**

**Fig.19**

**Fig.20**

## Fig.21

## Fig.22A

Time elapsed since the stop of ultraviolet irradiation
by low-pressure mercury vapor lamp [h]

**Fig.22B**

Time elapsed since the stop of ultraviolet irradiation
by KrCl excimer lamp [h]

**Fig.23**

Absorbance spectrum of E.coli

<div align="center">**INTERNATIONAL SEARCH REPORT**</div>

| International application No. |
|---|
| **PCT/JP2022/002380** |

**A.    CLASSIFICATION OF SUBJECT MATTER**

*A61L 2/10*(2006.01)i; *A61L 9/20*(2006.01)i
FI:    A61L2/10; A61L9/20

According to International Patent Classification (IPC) or to both national classification and IPC

**B.    FIELDS SEARCHED**

Minimum documentation searched (classification system followed by classification symbols)

A61L2/10; A61L9/20

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| Y | JP 2018-517488 A (THE TRUSTEES OF COLUMBIA UNIVERSITY IN THE CITY OF NEW YORK) 05 July 2018 (2018-07-05) claims 1, 5-7, paragraphs [0040], [0048]-[0049] | 1-27 |
| Y | JP 2018-130131 A (ENEFOREST KK) 23 August 2018 (2018-08-23) paragraphs [0074], [0086], [0089], [0091]-[0092], [0101], [0134] | 1-11, 24-27 |
| Y | JP 2017-528258 A (DAYLIGHT MEDICAL, INC.) 28 September 2017 (2017-09-28) paragraphs [0015], [0017], [0033], fig. 2 | 1-2, 24-27 |
| Y | US 2017/0246329 A1 (LLOYD, Ralph Birchard) 31 August 2017 (2017-08-31) claim 1, paragraphs [0097]-[0098], [0121], fig. 6-7, 12 | 1, 12-14 |
| Y | JP 2016-193059 A (TOKUYAMA CORP) 17 November 2016 (2016-11-17) claims | 14 |
| Y | JP 2019-536492 A (BRAINLIT AB) 19 December 2019 (2019-12-19) paragraphs [0031], [0042], [0103] | 1, 15-23 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 March 2022** | **12 April 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/002380**

**C.    DOCUMENTS CONSIDERED TO BE RELEVANT**

| Category* | Citation of document, with indication, where appropriate, of the relevant passages | Relevant to claim No. |
|---|---|---|
| E, X | WO 2022/024587 A1 (MINEBEA MITSUMI INC.) 03 February 2022 (2022-02-03) claims 1-4 | 1 |
| E, X | JP 2022-47676 A (MINEBEA MITSUMI INC.) 25 March 2022 (2022-03-25) claims 1-9, paragraphs [0014]-[0015], [0042]-[0043] | 1, 3, 13, 24-25 |

Form PCT/ISA/210 (second sheet) (January 2015)

**INTERNATIONAL SEARCH REPORT**
**Information on patent family members**

International application No.

**PCT/JP2022/002380**

| Patent document cited in search report | | | Publication date (day/month/year) | Patent family member(s) | | | Publication date (day/month/year) |
|---|---|---|---|---|---|---|---|
| JP | 2018-517488 | A | 05 July 2018 | US | 2018/0169279 | A1 | |
| | | | | claims, paragraphs [0060], [0067]-[0069] | | | |
| | | | | US | 2020/0085984 | A1 | |
| | | | | US | 2020/0215215 | A1 | |
| | | | | US | 2020/0306397 | A1 | |
| | | | | US | 2020/0353112 | A1 | |
| | | | | WO | 2016/196904 | A1 | |
| | | | | EP | 3302328 | A1 | |
| | | | | CN | 107613895 | A | |
| | | | | CN | 111888514 | A | |
| JP | 2018-130131 | A | 23 August 2018 | (Family: none) | | | |
| JP | 2017-528258 | A | 28 September 2017 | US | 2016/0317690 | A1 | |
| | | | | paragraphs [0019], [0021], [0037], fig. 2 | | | |
| | | | | US | 2018/0055964 | A1 | |
| | | | | US | 2019/0321504 | A1 | |
| | | | | WO | 2016/049143 | A1 | |
| | | | | EP | 3197505 | A1 | |
| | | | | AU | 2015320781 | A | |
| | | | | AU | 2018208755 | A | |
| US | 2017/0246329 | A1 | 31 August 2017 | US | 2020/0254125 | A1 | |
| | | | | US | 2017/0246331 | A1 | |
| | | | | WO | 2017/147433 | A1 | |
| | | | | WO | 2017/147460 | A1 | |
| JP | 2016-193059 | A | 17 November 2016 | US | 2018/0110890 | A1 | |
| | | | | claims | | | |
| | | | | WO | 2016/158524 | A1 | |
| | | | | TW | 201701907 | A | |
| | | | | CN | 107405415 | A | |
| | | | | KR | 10-2017-0134969 | A | |
| JP | 2019-536492 | A | 19 December 2019 | US | 2019/0192710 | A1 | |
| | | | | paragraphs [0031], [0042], [0103] | | | |
| | | | | WO | 2018/041986 | A1 | |
| | | | | EP | 3290058 | A1 | |
| | | | | EP | 3506953 | A1 | |
| | | | | CN | 109641075 | A | |
| | | | | KR | 10-2019-0075904 | A | |
| | | | | RU | 2019107730 | A | |
| WO | 2022/024587 | A1 | 03 February 2022 | (Family: none) | | | |
| JP | 2022-47676 | A | 25 March 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018130535 A

- US 10588993 B